# EUROPEAN PATENT APPLICATION

(11) **EP 4 134 133 A1**
(43) Date of publication of application: **15.02.2023**
(21) Application number: 22183027.6
(22) Date of filing: 30.07.2019
(51) Int. Cl.: A61P 21/00, A61K 38/16, A61K 47/60, C07K 14/33

(54) **PEGYLATED TETANUS NEUROTOXINS AND TREATMENT OF HYPOTONIA**

(30) Priority: 31.07.2018 AU 2018902779
(62) Divisional of application: 19843147.0
(71) Applicant: Snoretox Pty Ltd, Kew VIC 3101 (AU)
(72) Inventor: MCLEAN, Thomas, Melbourne, Victoria, 3000 (AU); SMOOKER, Peter, Melbourne, Victoria, 3000 (AU); NORBURY, Luke, Melbourne, Victoria, 3000 (AU); COLOE, Peter, Melbourne, Victoria, 3000 (AU); CONDUIT, Russell, Melbourne, Victoria, 3000 (AU); SASSE, Anthony, Kew, Victoria, 3101 (AU)
(74) Representative: Kolster Oy Ab

(57) **Abstract**

The invention relates to a composition comprising a first PEGylated tetanus neurotoxin (PEG-TeNT) comprising tetanus neurotoxin (TeNT) conjugated to polyethylene glycol (PEG) and a second TeNT. The invention also relates to various PEG-TeNTs. The invention also relates to a method of treating hypotonia using the composition or various PEG-TeNTs, and a kit comprising the composition or various PEG-TeNTs. In one embodiment, the hypotonia is obstructive sleep apnoea.

## Description

### FIELD

The invention relates to a composition comprising a first PEGylated tetanus neurotoxin (PEG-TeNT) comprising tetanus neurotoxin (TeNT) conjugated to polyethylene glycol (PEG) and a second TeNT. The invention also relates to a method of treating hypotonia using the composition.

### BACKGROUND

Tetanus neurotoxin (TeNT) is produced by *Clostridium tetani.* TeNT acts at the spinal cord and blocks release at the spinal inhibitory interneurons of γ-aminobutyric acid (GABA) and glycine, which are inhibitory neurotransmitters. As such, TeNT causes spastic paralysis. TeNT does not occur in multiple serotypes.

Exploitation of biological properties of TeNT, or at least fragments of TeNT, for therapy has been proposed. However, long-term treatment with protein therapeutic agents tends to lead to a targeted immune response. As there is only one serotype of TeNT known, serotype switching to circumvent immunity is not an option for TeNT-based therapy. Moreover, many populations are vaccinated against TeNT, thereby precluding TeNT-based therapy.

US 2002/0197278 A1 discloses use of PEGylated botulinum toxins for treating disorders of inappropriate muscle contraction. US 2002/0197278 A1 also suggests use of PEGylated TeNT for treating disorders of inappropriate muscle contraction, e.g. migration headache or strabismus. However, as noted above, TeNT causes muscle contraction, thereby precluding its use, PEGylated or not, for treating disorders of inappropriate muscle contraction.

Wan et al. Process Biochemistry (2017) 52: 183-191 discloses the effect of PEGylation on the anti-PEG immune response resulting from administration of PEGylated proteins, but does not exploit its findings for any therapy.

WO 2016/001762 A1 discloses use of a PEGylated TeNT fragment c (c) for increasing muscle mass. Fragment c (50 kDa) is generated when TeNT is enzymatically cleaved by papain and corresponds to the 451 amino acids at the C-terminus of the TeNT heavy chain. Fragment c retains the binding, internalization and trans-synaptic transport capabilities of undigested TeNT, but does not disrupt any neuronal processes, and is therefore nontoxic.

A need exists for a TeNT-based therapy that avoids the pre-existing anti-TeNT immune response in tetanus toxoid immunised subjects.

It is to be understood that if any prior art publication is referred to herein, such reference does not constitute an admission that the publication forms a part of the common general knowledge in the art in Australia or any other country.

### SUMMARY

The inventors have appreciated that exploitation of TeNT has not been fully realised because of the adaptive immune system, which upon administration of a protein therapeutic agent, produces an antibody response, thereby decreasing efficacy of the protein therapeutic agent. The adaptive immune response may be intentional, as a result of vaccination, as demonstrated by many populations that have been immunised against TeNT. Alternatively, the adaptive immune response may be unintentional, resulting from repeated exposure to the protein therapeutic agent.

The inventors have produced a family of modified TeNTs and a treatment regimen that address these problems. Specifically, the invention provides a family of PEGylated TeNTs (PEG-TeNTs) that each evade the immune system, and a tiered treatment regimen in which a different, replacement PEG-TeNT is used for treatment when efficacy of a previously administered PEG-TeNT decreases or when a patient's immunological profile precludes the use of another PEG-TeNT.

A first aspect provides a tetanus neurotoxin (TeNT) or fragment thereof, comprising one or more surface serine to cysteine amino acid substitutions relative to SEQ ID NO: 1.

In one embodiment of the first aspect, the substituted cysteine is conjugated to polyethylene glycol (PEG).

A second aspect provides a TeNT or fragment thereof comprising, relative to SEQ ID NO: 1: R1225K; R1225E; W1288A; W1288Y; W1288F; W1288L; R1225K and W1288A; R1225E and W1288A; R1225K and W1288Y; R1225E and W1288Y; R1225K and W1288F; R1225E and W1288F; R1225K and W1288L; R1225E and W1288L; R1225del; W1288del; or R1225del and W1288del; E270A; Y374A; E270A and Y374; G270del; Y374del; or a combination thereof, wherein the TeNT or fragment is inactive.

A third aspect provides a composition comprising: (i) a first PEGylated tetanus neurotoxin (PEG-TeNT) comprising tetanus neurotoxin (TeNT) conjugated to polyethylene glycol (PEG); and (ii) a second TeNT, wherein the first PEG-TeNT is not conjugated to the second TeNT.

A fourth aspect provides a method for treating hypotonia, the method comprising administering to a subject: (i) a first PEGylated tetanus neurotoxin (PEG-TeNT) comprising tetanus neurotoxin (TeNT) conjugated to polyethylene glycol (PEG); and (ii) a second TeNT.

The fourth aspect also provides use of a first PEGylated tetanus neurotoxin (PEG-TeNT) comprising a tetanus neurotoxin (TeNT) conjugated to polyethylene glycol (PEG) in the manufacture of a medicament for treating hypotonia in a subject administered a second TeNT.

The fourth aspect also provides use of a second tetanus neurotoxin (TeNT) in the manufacture of a medicament for treating hypotonia in a subject administered a first PEGylated tetanus neurotoxin (PEG-TeNT) comprising a tetanus neurotoxin (TeNT) conjugated to polyethylene glycol (PEG).

The fourth aspect also provides use of: (i) a first PEGylated tetanus neurotoxin (PEG-TeNT) comprising a tetanus neurotoxin (TeNT) conjugated to polyethylene glycol (PEG); and (ii) a second TeNT in the manufacture of a medicament for treating hypotonia.

The fourth aspect also provides a first PEGylated tetanus neurotoxin (PEG-TeNT) comprising a tetanus neurotoxin (TeNT) conjugated to polyethylene glycol (PEG) for use in a method for treating hypotonia in a subject administered a second TeNT.

The fourth aspect also provides a second tetanus neurotoxin (TeNT) for use in a method for treating hypotonia in a subject administered a first PEGylated tetanus neurotoxin (PEG-TeNT) comprising a tetanus neurotoxin (TeNT) conjugated to polyethylene glycol (PEG).

The fourth aspect also provides: (i) a first PEGylated tetanus neurotoxin (PEG-TeNT) comprising a tetanus neurotoxin (TeNT) conjugated to polyethylene glycol (PEG); and (ii) a second TeNT for use in a method for treating hypotonia.

In embodiments of the fourth aspect, a composition may comprise the first PEG-TeNT and the second TeNT.

The fourth aspect also provides a composition comprising: (i) a first PEGylated tetanus neurotoxin (PEG-TeNT) comprising tetanus neurotoxin (TeNT) conjugated to polyethylene glycol (PEG); and (ii) a second TeNT for use in a method for treating hypotonia.

In one embodiment, the first PEG-TeNT or the second TeNT comprises a PEGylated TeNT light chain (LC), a PEGylated TeNT heavy chain (HC), a PEGylated TeNT heavy chain (HC) and PEGylated TeNT light chain (LC), or a PEGylated TeNT fragment c (c). In one embodiment, the first PEG-TeNT or the second TeNT comprises PEG-TeNT-LC-HC.

In another embodiment, the first PEG-TeNT or the second TeNT is PEG-TeNT-HC comprising a PEGylated HC. In this embodiment, LC is not PEGylated. In another embodiment, the first PEG-TeNT or the second TeNT is PEG-TeNT-LC-c comprising a PEGylated LC and a PEGylated c. In this embodiment, HN is not PEGylated.

In a further embodiment, the first PEG-TeNT is PEG-TeNT-HC, and the second TeNT is PEG-TeNT-LC-c.

In one embodiment, the second TeNT comprises an inactivated TeNT. Relative to SEQ ID NO: 1, the inactivated TeNT may comprise: R1225K; R1225E; W1288A; W1288Y; W1288F; W1288L; R1225K and W1288A; R1225E and W1288A; R1225K and W1288Y; R1225E and W1288Y; R1225K and W1288F; R1225E and W1288F; R1225K and W1288L; R1225E and W1288L; R1225del; W1288del; R1225del or W1288del; E270A; Y374A; E270A and Y374A; G270del; Y374del; or a combination thereof. In one embodiment, the second TeNT comprises an inactivated TeNT comprising R1225E and W1288A.

In one embodiment, the subject is administered: a PEG-TeNT comprising PEGylated c (PEG-TeNT-c) until efficacy decreases; then a composition comprising PEG-TeNT-HC and PEG-TeNT-LC-c until efficacy decreases; then a PEG-TeNT comprising a PEGylated LC and a PEGylated HC (PEG-TeNT-LC-HC).

In one embodiment, treating comprises adminstering to the subject: a PEG-TeNT comprising PEGylated c (PEG-TeNT-c); then a composition comprising PEG-TeNT-HC and PEG-TeNT-LC-c; then a PEG-TeNT comprising a PEGylated LC and a PEGylated HC (PEG-TeNT-LC-HC), to determine the immunological profile of anti-TeNT antibodies of the subject and determine the effective composition of PEG-TeNTs based on that profile.

In one embodiment, the hypotonia is obstructive sleep apnoea.

In another embodiment, the composition is a therapeutic composition. In another embodiment, the composition is a cosmetic composition.

Also disclosed is a method for preparing a protein consisting of TeNT LC and TeNT HN (LC-HN), the method comprising expressing in a host cell a nucleic acid molecule encoding an amino acid comprising SEQ ID NO: 6.

A fifth aspect provides a PEGylated tetanus neurotoxin (PEG-TeNT), comprising tetanus neurotoxin (TeNT) conjugated to polyethylene glycol (PEG).

In one embodiment, the TeNT light chain (LC) is PEGylated, the TeNT heavy chain (HC) is PEGylated, or the TeNT fragment c (c) is PEGylated. In one embodiment, LC is PEGylated and HC is PEGylated (PEG-TeNT-LC-HC), or LC is PEGylated and c is PEGylated (PEG-TeNT-LC-c) .

In another embodiment, PEG is conjugated to a lysine residue of TeNT. In another embodiment, PEG is conjugated to a cysteine residue of TeNT. In one embodiment, PEG is conjugated to a cysteine residue of TeNT, wherein the cysteine residue is either native or a substitution for a serine residue relative to SEQ ID NO: 1.

In one embodiment, the PEG has a molecular weight of about 2 kDa, about 5 kDa, about 10 kDa, or about 20 kDa, or about 30 kDa.

A sixth aspect provides a method for treating hypotonia, the method comprising administering to a subject the PEG-TeNT of the fifth aspect, wherein the PEG-TeNT is not conjugated to a second TeNT.

The sixth aspect also provides use of the PEG-TeNT of the fifth aspect in the manufacture of a medicament for treating hypotonia, wherein the PEG-TeNT is not conjugated to a second TeNT.

Alternatively, the sixth aspect provides the PEG-TeNT of the fifth aspect for use in a method for treating hypotonia, the method comprising administering to a subject the PEG-TeNT, wherein the PEG-TeNT is not conjugated to a second TeNT.

In one embodiment, the subject is administered a first PEG-TeNT comprising a PEGylated HC (PEG-TeNT-HC) and a second PEG-TeNT comprising a PEGylated LC and a PEGylated c (PEG-TeNT-LC-c).

In another embodiment, the subject is administered: a PEG-TeNT comprising a PEGylated c (PEG-TeNT-c); and/or a first PEG-TeNT comprising a PEGylated HC (PEG-TeNT-HC) and a second PEG-TeNT comprising a PEGylated LC-c (PEG-TeNT-LC-c); and/or a PEG-TeNT comprising a PEGylated HC and a PEGylated LC (PEG-TeNT-LC-HC).

In one embodiment, treating comprises administering to the subject: a PEG-TeNT comprising PEGylated c (PEG-TeNT-c) until efficacy decreases. Thereafter, treating may comprise administering to the subject: a PEG-TeNT-HC and a PEG-TeNT-LC-c until efficacy decreases. Thereafter, treating may comprise administering to the subject: a PEG-TeNT comprising a PEGylated LC and a PEGylated HC (PEG-TeNT-LC-HC).

In one embodiment, treating comprises administering to the subject: a PEG-TeNT comprising PEGylated c (PEG-TeNT-c) until efficacy decreases; then a PEG-TeNT-HC and a PEG-TeNT-LC-c until efficacy decreases; then a PEG-TeNT comprising a PEGylated LC and a PEGylated HC (PEG-TeNT-LC-HC).

In one embodiment, treating comprises administering to the subject: a PEG-TeNT comprising PEGylated c (PEG-TeNT-c) comprising PEG having a molecular weight of about 5 kDa until efficacy decreases; then a PEG-TeNT comprising PEGylated c (PEG-TeNT-c) comprising PEG having a molecular weight of about 10 kDa until efficacy decreases; then a PEG-TeNT comprising PEGylated c (PEG-TeNT-c) comprising PEG having a molecular weight of about 20 kDa until efficacy decreases. Thereafter, treating may comprise administering to the subject: a PEG-TeNT-HC and a PEG-TeNT-LC-c, either or both of which comprise PEG having a molecular weight of 5 kDa, until efficacy decreases; then a PEG-TeNT-HC and a PEG-TeNT-LC-c, either or both of which comprise PEG having a molecular weight of 10 kDa, until efficacy decreases; then a PEG-TeNT-HC and a PEG-TeNT-LC-c, either or both of which comprise PEG having a molecular weight of 20 kDa, until efficacy decreases. Thereafter, treating may comprise administering to the subject: a PEG-TeNT comprising a PEGylated LC and a PEGylated HC (PEG-TeNT-LC-HC), either or both of which comprise PEG having a molecular weight of 5 kDa, until efficacy decreases; then a PEG-TeNT comprising a PEGylated LC and a PEGylated HC (PEG-TeNT-LC-HC), either or both of which comprise PEG having a molecular weight of 10 kDa, until efficacy decreases; then a PEG-TeNT comprising a PEGylated LC and a PEGylated HC (PEG-TeNT-LC-HC), either or both of which comprise PEG having a molecular weight of 20 kDa.

In one embodiment, treating comprises adminstering to the subject: a PEG-TeNT comprising PEGylated c (PEG-TeNT-c); then a composition comprising PEG-TeNT-HC and PEG-TeNT-LC-c; then a PEG-TeNT comprising a PEGylated LC and a PEGylated HC (PEG-TeNT-LC-HC), to determine the immunological profile of anti-TeNT antibodies of the subject and determine the effective composition of PEG-TeNTs based on that profile.

In another embodiment, treating with a TeNT further comprises administering an inactivated TeNT. Relative to SEQ ID NO: 1, the inactivated TeNT may comprise: R1225K; R1225E; W1228A; W1288Y; W1288F; W1288L; R1225K and W1288A; R1225E and W1288A; R1225K and W1288Y; R1225E and W1288Y; R1225K and W1288F; R1225E and W1288F; R1225K and W1288L; R1225E and W1288L; R1225del; W1288del; R1225del or W1288del. In one embodiment, the second TeNT comprises an inactivated TeNT comprising R1225E and W1288A; E270A; Y374A; E270A and Y374A; G270del; Y374del; or a combination thereof.

In one embodiment, the hypotonia is obstructive sleep apnoea.

A seventh aspect provides a kit comprising the TeNT of the first aspect, the composition of the second aspect or the PEG-TeNT of the fifth aspect.

In one embodiment, the composition or PEG-TeNT is used according to the method of the third or sixth aspect, respectively.

### BRIEF DESCRIPTION OF THE FIGURES

Figure 1 is a schematic representation of example PEG-TeNTs of the invention: A PEG-TeNT-c; B PEG-TeNT-HC, C PEG-TeNT-LC-c, D PEG-TeNT-LC-HC, where the TeNT is entire and active, i.e. the TeNT comprises both chains, and PEGylation is present on specific regions, i.e. c, HC, LC-c, or LC-HC, respectively.
Figure 2 is the amino acid sequence (SEQ ID NO: 1) of mature TeNT comprising 1314 amino acids.
Figure 3 is a nucleic acid sequence (SEQ ID NO: 2) of the vector pRSET-TeNT encoding TeNT.
Figure 4 is a map of the vector pRSET-TeNT encoding TeNT. TeNT is expressed with an N-terminal His₆-tag. The nucleic acid was inserted within the MCS of the pRSET-A vector and expressed under the control of a T7 promoter.
Figure 5 is the amino acid sequence (SEQ ID NO: 3) of HC comprising amino acids 457 to 1314 of SEQ ID NO: 1.
Figure 6 is the amino acid sequence (SEQ ID NO: 4) of c comprising amino acids 864 to 1314 of SEQ ID NO: 1.
Figure 7 is a nucleic acid sequence (SEQ ID NO: 5) of the vector pRSET-TeNT-c encoding c.
Figure 8 is a map of the vector pRSET-TeNT-c encoding c. c is expressed with an N-terminal His₆-tag. The nucleic acid was inserted within the MCS of the pRSET-A vector and expressed under the control of a T7 promoter.
Figure 9 is the amino acid sequence (SEQ ID NO: 6) of LC-HN comprising amino acids 1 to 863 of SEQ ID NO: 1.
Figure 10 is a nucleic acid sequence (SEQ ID NO: 7) of the vector pRSET-TeNT-LC-HN encoding LC-HN.
Figure 11 is a map of the vector pRSET-TeNT-LC-HN encoding LC-HN. LC-HN is expressed with an N-terminal His₆-tag. The nucleic acid was inserted within the MCS of the pRSET-A vector and expressed under the control of a T7 promoter.
Figure 12 is an amino acid sequence (SEQ ID NO: 8) of a non-functional c comprising amino acid substitutions W1288A and R1225E relative to SEQ ID NO: 1.
Figure 13 is a nucleic acid sequence (SEQ ID NO: 9) of the vector pRSET-TeNT-c encoding the non-functional c of Figure 12 (SEQ ID NO: 8) comprising amino acid substitutions W1288A and R1225E relative to SEQ ID NO: 1.
Figure 14 is a map of the vector pRSET-TeNT-c encoding the non-functional c of Figure 12 (SEQ ID NO: 8) comprising amino acid substitutions W1288A and R1225E relative to SEQ ID NO: 1. c, comprising amino acid substitutions W1288A and R1225E relative to SEQ ID NO: 1, is expressed with an N-terminal His₆-tag. The nucleic acid was inserted within the MCS of the pRSET-A vector and expressed under the control of a T7 promoter.
Figure 15 is an amino acid sequence (SEQ ID NO: 10) of a non-functional TeNT comprising amino acid substitutions W1288A and R1225E relative to SEQ ID NO: 1.
Figure 16 is a nucleic acid sequence (SEQ ID NO: 11) of the vector pRSET-TeNT encoding the non-functional TeNT of Figure 15 (SEQ ID NO: 10) comprising amino acid substitutions W1288A and R1225E relative to SEQ ID NO: 1.
Figure 17 is a map of the vector pRSET-TeNT encoding the non-functional TeNT of Figure 15 (SEQ ID NO: 10) comprising amino acid substitutions W1288A and R1225E relative to SEQ ID NO: 1. TeNT, comprising amino acid substitutions W1288A and R1225E relative to SEQ ID NO: 1, is expressed with an N-terminal His₆-tag. The nucleic acid was inserted within the MCS of the pRSET-A vector and expressed under the control of a T7 promoter.
Figure 18 is the amino acid sequence (SEQ ID NO: 12) of a mature 1314 amino acid TeNT comprising surface serine to cysteine amino acid substitutions S81C, S120C, S144C, S248C, S335C, S428C, S600C, S963C, S1041C, S1155C, and S1187C relative to SEQ ID NO: 1.
Figure 19 is a nucleic acid sequence (SEQ ID NO: 13) of the vector pRSET-TeNT encoding the surface serine to cysteine substituted mature TeNT of Figure 18 (SEQ ID NO: 12).
Figure 20 is a map of the vector pRSET-TeNT of Figure 19 (SEQ ID NO: 13) encoding the surface serine to cysteine substituted mature TeNT of Figure 18 (SEQ ID NO: 12). The surface serine to cysteine substituted TeNT is expressed with an N-terminal His₆-tag. The nucleic acid was inserted within the MCS of the pRSET-A vector and expressed under the control of a T7 promoter.
Figure 21 is the amino acid sequence (SEQ ID NO: 14) of a mature 1314 amino acid TeNT comprising surface serine to cysteine amino acid substitutions S81C, S120C, S144C, S248C, S335C, S428C, S963C, S1041C, S1155C, and S1187C, relative to SEQ ID NO: 1, in the LC and c regions.
Figure 22 is a nucleic acid sequence (SEQ ID NO: 15) of the vector pRSET-TeNT encoding the surface serine to cysteine substituted mature TeNT of Figure 21 (SEQ ID NO: 14).
Figure 23 is a map of the vector pRSET-TeNT of Figure 22 (SEQ ID NO: 15) encoding the surface serine to cysteine substituted mature TeNT of Figure 21 (SEQ ID NO: 14). The surface serine to cysteine substituted TeNT is expressed with an N-terminal His₆-tag. The nucleic acid was inserted within the MCS of the pRSET-A vector and expressed under the control of a T7 promoter.
Figure 24 is the amino acid sequence (SEQ ID NO: 16) of a TeNT comprising HC surface serine to cysteine amino acid substitutions S600C, S963C, S1041C, S1155C, and S1187C relative to SEQ ID NO: 1.
Figure 25 is a nucleic acid sequence (SEQ ID NO: 17) of the vector pRSET-TeNT encoding the surface serine to cysteine substituted mature TeNT of Figure 24 (SEQ ID NO: 16).
Figure 26 is a map of the vector pRSET-TeNT of Figure 25 (SEQ ID NO: 17) encoding the surface serine to cysteine substituted TeNT of Figure 24 (SEQ ID NO: 16). The surface serine to cysteine substituted TeNT is expressed with an N-terminal His₆-tag. The nucleic acid was inserted within the MCS of the pRSET-A vector and expressed under the control of a T7 promoter.
Figure 27 is a 3-dimensional protein structure model of TeNT derived from crystallography data deposited in the protein data bank (accession ID PDB: 5N0B) mapping epitopes recognised by major human antibody clonotypes, as identified by da Silva Antunes et al (2017) and Palermo et al (2017), onto the model using Discovery Studio. Surface serine residues in or around the identified epitopes were selected for mutation to cysteine for subsequent PEGylation.
Figure 28 comprises two photographs of SDS-PAGE analyses of PEG-TeNTs comprising PEGs of increasing molecular weight and detected (A) using Coomassie blue and (B) by Western blot using polyclonal anti-TeNT antibodies. (B) shows that immunogenicity is proportional to PEG molecular weight. Molecular weight marker lane 1 with kDa on left edge, TeNT lane 2, 2 kDa PEG-TeNT-HC-LC lane 3, 5 kDa PEG-TeNT-LC-HC lane 4, 10 kDa PEG-TeNT-LC-HC lane 5, 20 kDa PEG-TeNT-LC-HC lane 6.

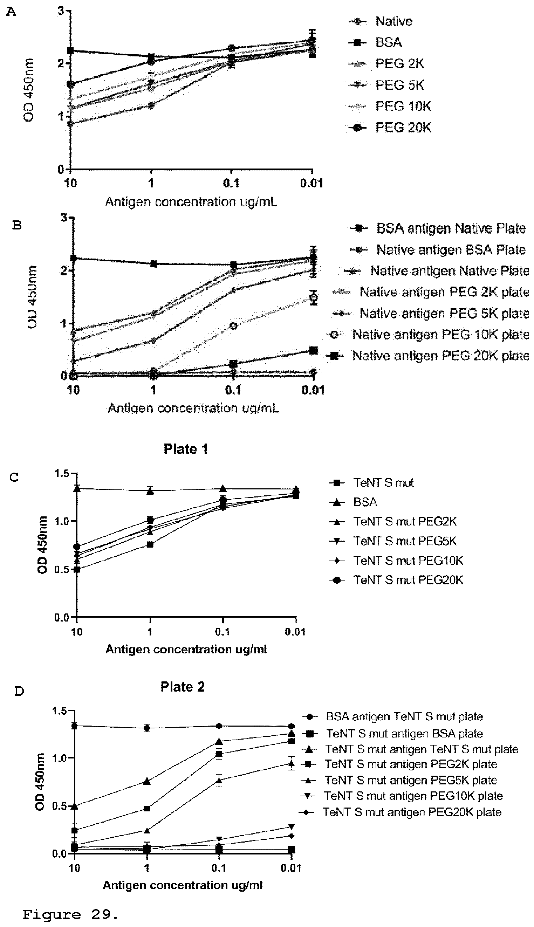

Figure 29 comprises four line graphs representing competitive ELISA assays. Four PEG-TeNTs and four PEG-TeNT-LC-c Serine mutants, each comprising a different molecular weight PEG (2 kDa, 5 kDa, 10 kDa and 20 kDa), were assayed against TeNT using a polyclonal anti-TeNT antibody. (A) TeNT was adsorbed to an ELISA plate and then probed with a polyclonal anti-TeNT antibody pre-incubated with each of four concentrations (10 µg/mL, 1 µg/mL, 0.1 µg/mL and 0.01 µg/mL) of four PEG-TeNT antigens (2 kDa, 5 kDa, 10 kDa and 20 kDa). (B) Each PEG-TeNT (2 kDa, 5 kDa, 10 kDa and 20 kDa) was adsorbed to a separate ELISA plate and then probed with a polyclonal anti-TeNT antibody pre-incubated with each of four concentrations (10 µg/mL, 1 µg/mL, 0.1 µg/mL and 0.01 µg/mL) of TeNT antigen. (C) TeNT-LC-c Serine mutant was adsorbed to an ELISA plate and then probed with a polyclonal anti-TeNT antibody pre-incubated with each of four concentrations (10 µg/mL, 1 µg/mL, 0.1 µg/mL and 0.01 µg/mL) of four PEG-TeNT-LC-c Serine mutant antigens (2 kDa, 5 kDa, 10 kDa and 20 kDa). (D) Each PEG-TeNT-LC-c Serine mutant (2 kDa, 5 kDa, 10 kDa and 20 kDa) was adsorbed to a separate ELISA plate and then probed with a polyclonal anti-TeNT antibody pre-incubated with each of four concentrations (10 µg/mL, 1 µg/mL, 0.1 µg/mL and 0.01 µg/mL) of TeNT-LC-c Serine mutant antigen.
Figure 30 comprises four photographs showing localised limb tetany in the hind limb of a female C57BL/6 mouse injected with PEG-TeNT-LC-HC. A and B show localised limb tetany in a naive mouse. C shows a lack of limb tetany after injection of 100 units of TeNT in a mouse vaccinated with tetanus toxoid, whereas D shows sustained limb tetany after injection of 80 units of PEG-TeNT LC-HC 20kDa in a mouse vaccinated with tetanus toxoid.
Figure 31 comprises two dot plots showing the clinical tetanus level exhibited in the hind leg of female C57BL/6 mice after injection with a defined number of units of TeNT, PEG-TeNT-LC-HC 20kDa, TeNT-LC-c Serine mutant, PEG-TeNT-LC-c Serine mutant 2kDa, or PEG-TeNT-LC-c Serine mutant 20kDa. A) Mice were injected with TeNT or PEG-TeNT-LC-HC 20kDa. B) Mice were injected with TeNT, TeNT-LC-c Serine mutant, PEG-TeNT-LC-c Serine mutant 2kDa or PEG-TeNT-LC-c Serine mutant 20kDa. One unit is the minimum amount of toxin required to produce stage four tetany within 24 hours in a naive mouse.
Figure 32 comprises two line graphs showing the progression of clinical tetany in mice vaccinated with tetanus toxoid. A shows the progression of tetany after injection with 4000 units of TeNT with or an without deactivated TeNT decoy. B shows the progression of tetany after injection with 40 units of TeNT-PEG 20kDa with or without deactivated TeNT decoy. In both cases the progression is clearly enhanced by the presence of decoy.

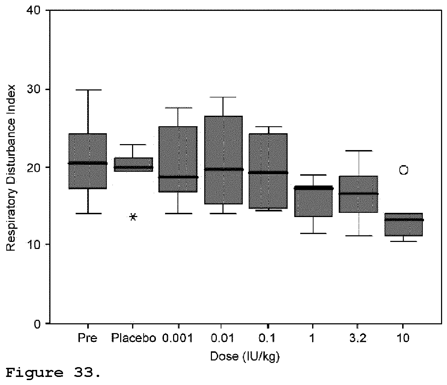

Figure 33 is a box plot showing the effect of increasing doses of tetanus toxin on the Respiratory Disturbance Index (RDI) of British Bulldogs treated according to example 23. The black bars represent the median RDI of the six studies at each dose of tetanus toxin. The shaded boxes represent the interquartile range and the brackets represent the minimum and maximum RDI sample at each dose. Increasing tetanus toxin was associated with a reduced RDI following the administration of 10 IU/kg compared with the RDI following administration of the placebo (P=0.043; Wilcoxon Signed Ranks test). Placebo and 10 IU/kg medians are additionally represented by o and *, respectively.

### DETAILED DESCRIPTION

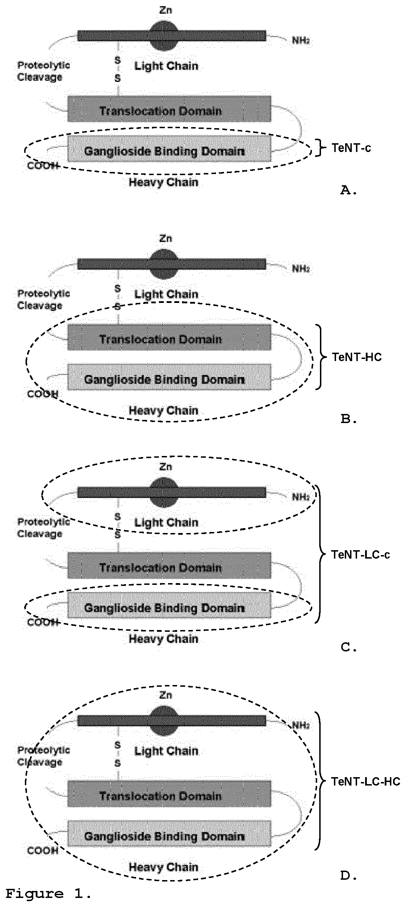

The present invention relates to immune-evading TeNT and PEG-TeNT molecules (Figure 1), compositions thereof, and their therapeutic and cosmetic use. In one embodiment, the invention relates to treating hypotonia, optionally obstructive sleep apnoea.

Described herein is use of a specifically modified TeNT and composition of TeNT and decoy (inactive TeNT) to treat hypotonia in subjects with a protective immune response against tetanus toxoid. To achieve that end, an active TeNT is modified by adding PEG, introducing specific mutations, or a combination thereof, for delivering a biologically active compound capable of increasing muscle tone in a tetanus-immune patient.

The activity can be demonstrated by the administration of a unit defined dose of modified toxin, or formulation, where at the same unit dose TeNT would exhibit no activity in a vaccinated subject. The introduction of specific surface mutations for the directed attachment of PEG molecules, based on the analysis of the three-dimensional structure of TeNT, allowed the masking of specific TeNT epitopes known to be targeted by the protective antibody response in vaccinated subjects. The combinations of PEGylation, site-directed mutation and decoy molecule formulations, greatly increased the effect of the molecule on increasing muscle tone in vaccinated mammalian models, relative to the equivalent units administered of TeNT.

US 2002/0197278 disclosed a series of PEGylated botulinum toxins for treating disorders of inappropriate muscle contraction and suggested that TeNT could be used as an alternative to botulium toxin. However, TeNT cannot be used to treat muscle contraction. Further, the alleged invention of US 2002/0197278 appears not to be enabled, because the methods disclosed do not include site directed masking of epitopes, and, to the best of the present inventors' knowledge, the three-dimensional structure and identification of epitopes required for deliberate masking of TeNT epitopes was not available at the priority date of US 2002/0197278.

Wan et *al.* is directed to the effect of PEGylation on the **anti-PEG** immune response resulting from administration of PEGylated proteins, but does not exploit its findings for any therapy. Although Wan et al. disclose that a PEGylated tetanus toxoid demonstrated reduced immunogenicity relative to non-PEGylated tetanus toxoid, Wan et *al.* do not present a therapeutically relevant molecule or formulation. Moreover, PEGylation of tetanus toxoid is not relevant to modification of active TeNT, because tetanus toxoid is a biologically inactive TeNT used for vaccination, which may be produced by formaldehyde cross-linking of TeNT. That is, tetanus toxoid, PEGylated or not, does not possess the combination of enzymatic, binding and translocational activity of active TeNT.

WO 2016/001762 A1 relates to a TeNT c-fragment alone, which is a molecule with no specific activity beyond binding neurotransmitters and entering the neuron.

Therefore, a need exists for a TeNT-based therapy that avoids the pre-existing anti-TeNT immunity in tetanus toxoid immunised subjects. Disclosed herein is a solution to this problem, in part provided by a masked, active and therapeutically relevant PEGylated TeNT.

### Tetanus neurotoxin (TeNT)

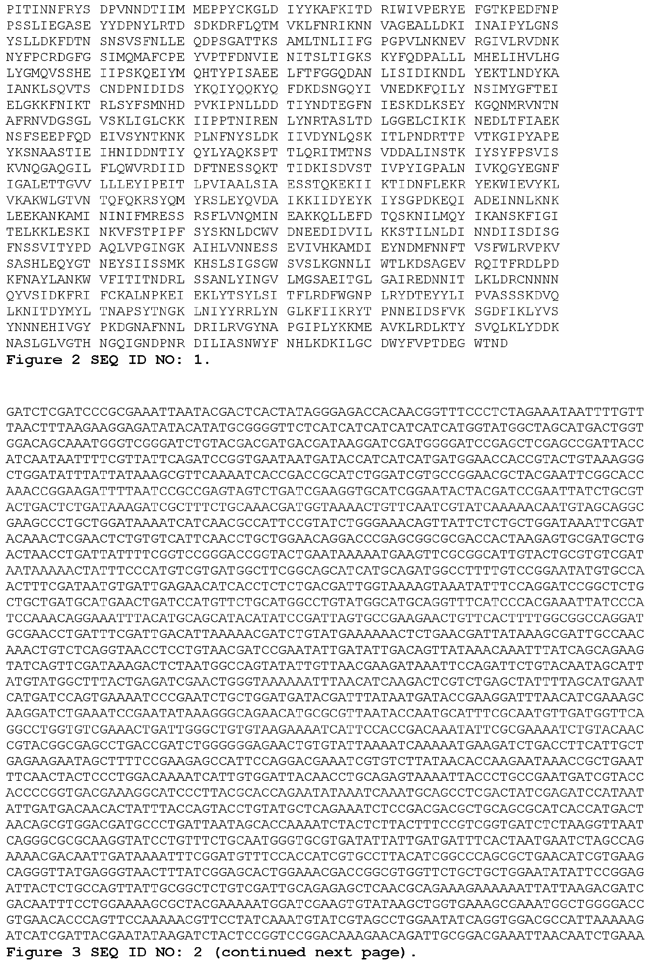

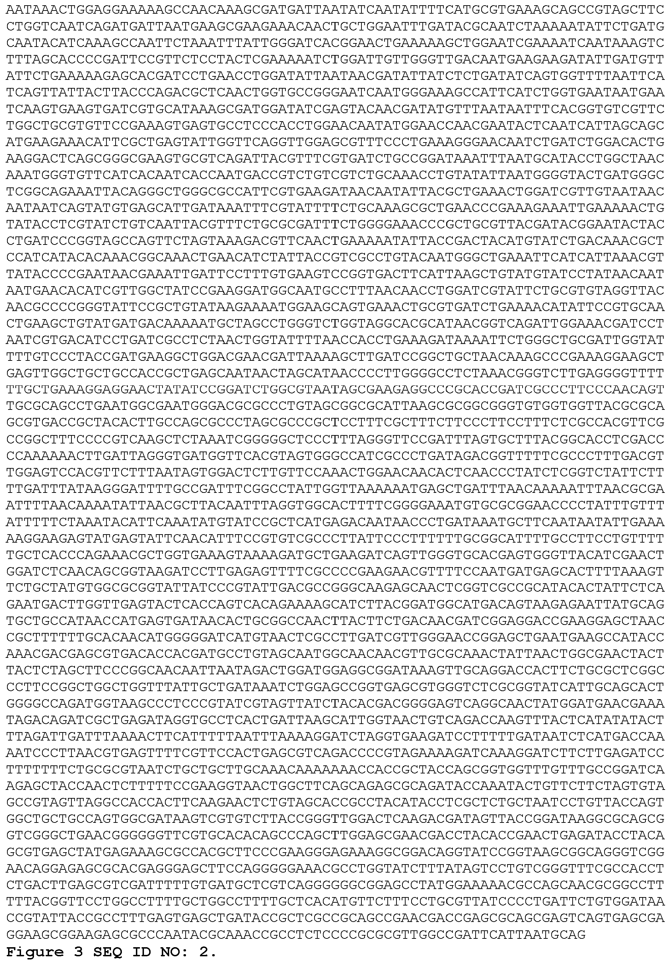

TeNT is approximately 150 kDa and is expressed from the *tetX* gene. A codon optimised nucleic acid sequence corresponding to the coding region of *tetX,* but lacking the initiator methionine codon, is provided in the vector sequence of Figure 3 (SEQ ID NO: 2). TeNT is expressed as one protein that is post-translationally cleaved - first to remove the initiator methionine and then into two parts: a 50kDa light chain (LC or A-chain) derived from the N-terminus of the uncleaved protein and a 100 kDa heavy chain (HC or B-chain) derived from the C-terminus of the uncleaved protein. The two chains are connected by an interchain disulfide bond, which is essential for neurotoxicity. The 1314 amino acid sequence of mature TeNT is provided in Figure 2 (SEQ ID NO: 1).

LC has zinc endopeptidase activity and attacks the vesicle-associated membrane protein (VAMP) that is necessary for vesicle fusion to membranes, thereby preventing neurotransmitter release.

Upon digestion with papain, HC can be cleaved into two domains, each of 50 kDa: an N-terminus translocation domain named HN; and a C-terminus ganglioside (membrane) binding domain named fragment c (c). TeNT lacking c is referred to herein as LC-HN.

c harbours two polysialoganglioside binding sites and binds to polysialogangliosides (GD2 GD1b, and GT1b) on the neuronal membrane. Thus, c mediates binding of TeNT to the presynaptic membrane of peripheral motor axons and aids movement of TeNT across that membrane into the neuron.

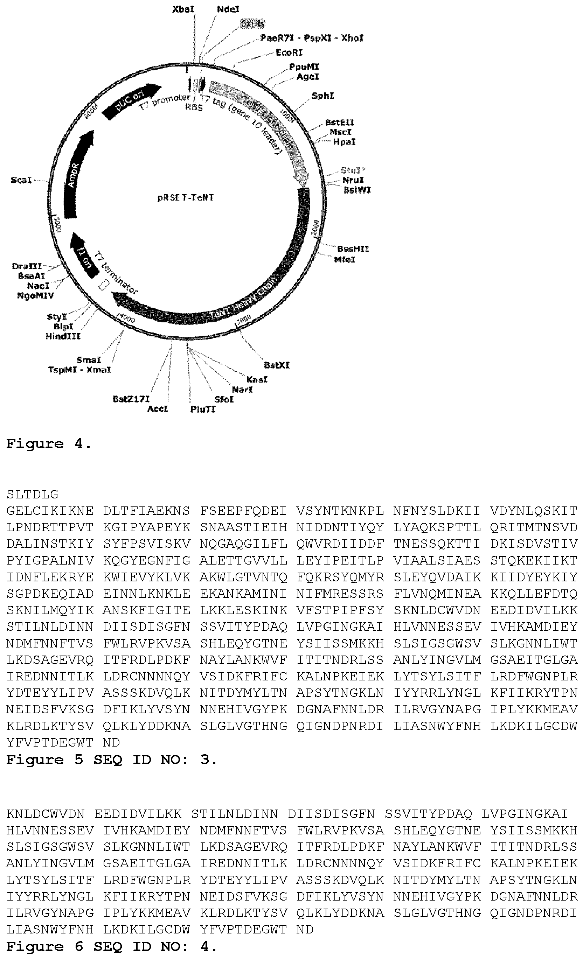

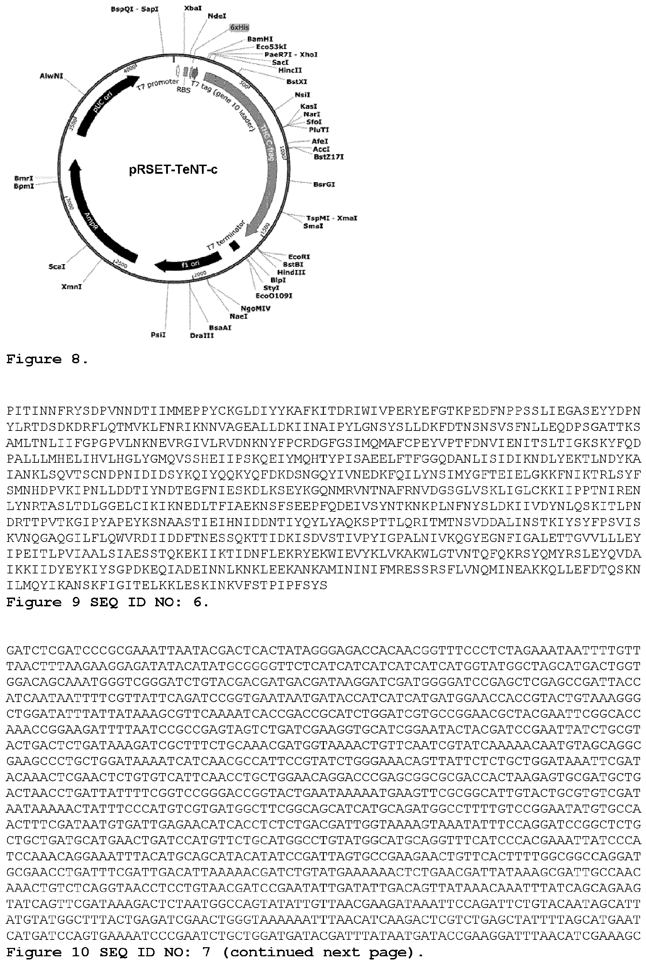

An amino acid sequence for:
TeNT lacking the initiator methionine is provided in Figure 2 (SEQ ID NO: 1);
HC is provided in Figure 5 (SEQ ID NO: 3);
c is provided in Figure 6 (SEQ ID NO: 4); and
LC-HN is provided in Figure 9 (SEQ ID NO: 6).

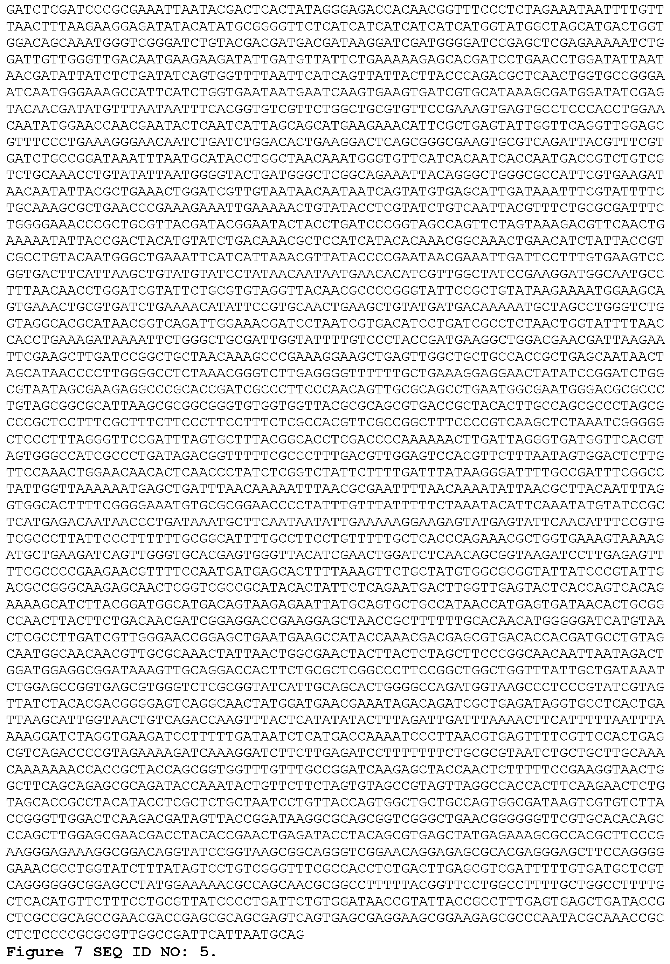

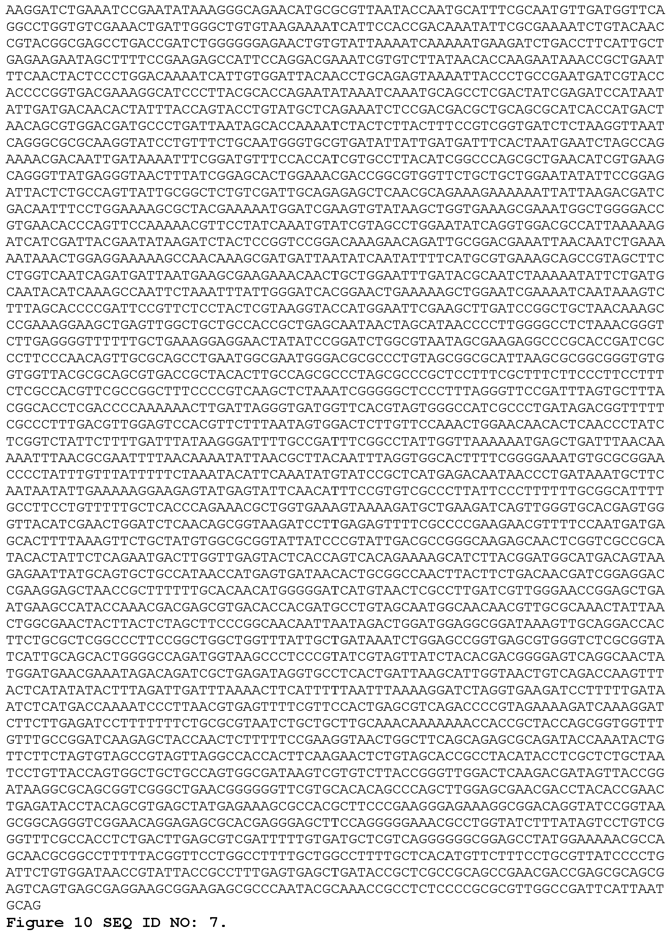

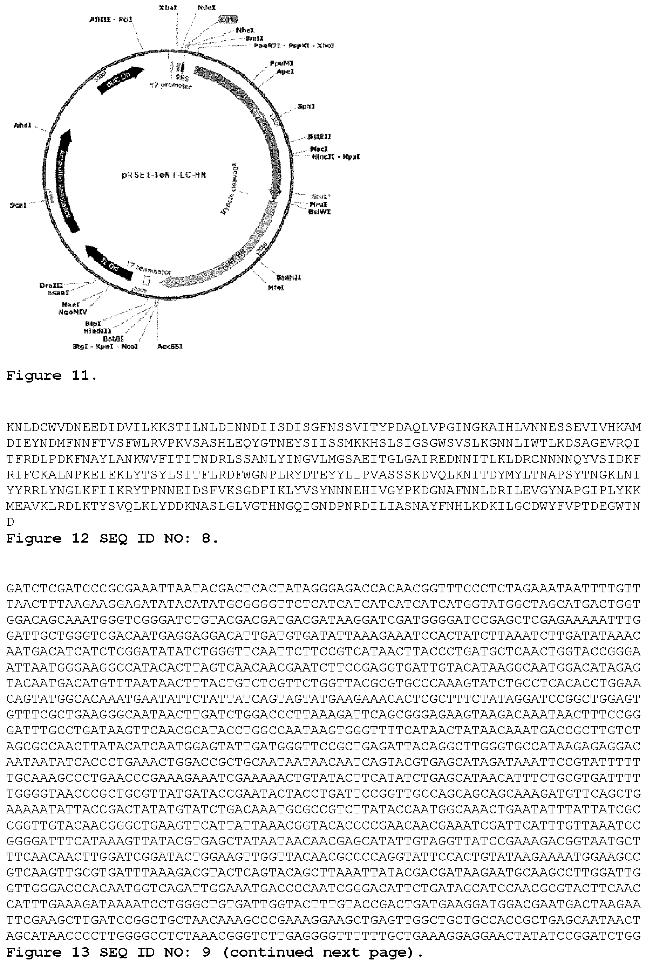

A codon-optimised nucleic acid sequence of a vector encoding and a vector map for:
c are provided in Figure 7 (SEQ ID NO: 5) and Figure 8; and
LC-HN are provided in Figure 10 (SEQ ID NO: 7) and Figure 11.

As used herein, "TeNT" is used in reference to a full TeNT molecule, consisting of the heavy chain and light chain. Subdomains and fragments are referred to herein by their abbreviations: light chain "LC"; heavy chain "HC"; heavy chain N-terminus domain "HN"; heavy chain fragment c "c"; light chain plus heavy chain N-terminus domain "LC-HN" (i.e. TeNT molecule lacking c). Where any subdomain or fragment is PEGylated, the prefix PEG is used: PEG-LC; PEG-HC; PEG-HN; PEG-c; PEG-LC-HN. In a full TeNT molecule comprising a PEGylated fragment or subdomain, the prefix PEG is used and the PEGylated subdomain or fragment is indicated: PEG-TeNT-LC; PEG-TeNT-HC; PEG-TeNT-LC-HC; PEG-TeNT-HN; PEG-TeNT-c; PEG-TeNT-LC-c; PEG-TeNT-LC-HN, and so on.

It will be appreciated that because of their specific functions, subdomains and fragents are not interchangeable for full TeNT.

TeNTs disclosed herein may be active or inactive. An active TeNT possesses the same biological activities of native TeNT. An inactive TeNT lacks one or more activities of native TeNT. In one embodiment, an inactive TeNT does not block release of inhibitory neurotransmitters. An inactive TeNT of the disclosure may also be referred to as a "decoy". In inactive TeNT includes tetanus toxoid. In one embodiment, an inactive TeNT is an inactive TeNT as disclosed herein.

In one embodiment, two or more TeNTs may be conjugated.

In another embodiment, TeNTs are not conjugated. In one embodiment of the compositions, methods and uses disclosed herein, a first PEG-TeNT is not conjugated to a second TeNT.

Immune-evading PEG-TeNTs disclosed herein and depicted in Figure 1 include:
TeNT with PEGylated fragment c (PEG-TeNT-c) (Figure 1A);
TeNT with PEGylated heavy chain (PEG-TeNT-HC) (Figure 1B);
TeNT with PEGylated light chain and PEGylated fragment c (PEG-TeNT-LC-c) (Figure 1C); and
TeNT fully PEGylated (PEG-TeNT-LC-HC) (Figure 1D).

PEG-TeNT-c advantageously evades the pre-existing immune response of the adaptive immune system in vaccinated subjects. In one embodiment, PEG-TeNT-c provides first tier treatment to be used until efficacy decreases.

PEG-TeNT-HC and PEG-TeNT-LC-c advantageously evade the pre-existing immune response of the adaptive immune system in vaccinated subjects and also evade the immune response of the adaptive immune system elicited by repeated exposure to PEG-TeNT-c.

In one embodiment, PEG-TeNT-HC and PEG-TeNT-LC-c together provide second tier treatment to be used until their efficacy decreases.

PEG-TeNT-LC-HC advantageously evades the pre-existing immune response of the adaptive immune system in vaccinated subjects and also evades the immune response of the adaptive immune system elicited by repeated exposure to PEG-TeNT-c and to PEG-TeNT-HC plus PEG-TeNT-LC-c.

In one embodiment, PEG-TeNT-LC-HC provides third tier treatment to be used until its efficacy decreases.

Also disclosed is TeNT with PEGylated light chain (PEG-TeNT-LC), TeNT with PEGylated LC and HN (PEG-TeNT-LC-HN), and TeNT with PEGylated HN (PEG-TeNT-HN).

The person skilled in the art will appreciate that the specific combinations of PEG-TeNTs and the order of treatment with those PEG-TeNT combinations may be altered.

### Polyethylene glycol (PEG)

PEG may be conjugated, for example, to lysine (e.g. amino-PEGylation), cysteine (e.g. thiol-PEGylation and bridging PEGylation), histidine, arginine, aspartic acid, asparagine (e.g. N-glyco-PEGylation), glutamic acid, glutamine (e.g. transglutaminase-mediated PEGylation), serine (e.g. O-glyco-PEGylation), threonine (e.g. O-glyco-PEGylation), or tyrosine residues in TeNT. Examples of PEGylation also include N-terminus PEGylation and C-terminus PEGylation.

PEGylation may be achieved by reacting PEG with a functional group that is hydroxyl-reactive, for example anhydrides, acid chlorides, chloroformates and carbonates. Alternatively, PEGylation may be achieved with functional groups such as aldehyde, ester, and amide.

PEG may be linear or branched.

PEG may be a modified PEG, for example, poly[oligo(ethylene glycol) methyl ether methacrylate] (POEGMA).

PEGylation may be site-specific PEGylation.

In one embodiment, surface serine residues of TeNT or a TeNT fragment are mutated to surface cysteine residues to facilitate directed PEG conjugation at immunogenic epitopes. In this context, a mutation is synonymous with substitution, for instance, a serine to cysteine substitution. Such mutations, or substitutions, include one or more, in any combination, of: S81C; S120C; S144C; S248C; S335C; S428C; S600C; S963C; S1041C; S1155C; and S1187C.

Functional groups for heterobifunctional PEGs include maleimide, vinyl sulfone, pyridyl disulfide, amine, carboxylic acid, and NHS ester.

In one embodiment, PEG is conjugated to a TeNT using carboxyl-to-amine crosslinking using the carbodiimide-EDC and sulfo-NHS.

The invention also contemplates a PEG-TeNT comprising different molecular weight PEGs conjugated to different subdomains or fragments of TeNT.

PEG may be conjugated or attached to TeNTs of the disclosure between 4°C and 25 °C for between 2 and 6 hours, for example. In one embodiment, PEG was conjugated to TeNT at room temperature for 6 hours.

### Indications

As used herein, "hypotonia" refers to any disorder comprising involuntary muscle weakness that may be treated by inhibiting inhibitory neurotransmitters, for instance GABA or glycine. As such, "hypotonia" includes reduced muscle tone secondary to reduced neurological drive or other causes and conditions of reduced or inadequate muscle tone, strength or neurological drive. Thus, in one embodiment, hypotonia may be neurological hypotonia.

Hypotonia disorders that may be treated with a PEG-TeNT, a composition or method according to the disclosure include obstructive sleep apnoea, apnoea, snoring, scoliosis, strabismus caused by muscle relaxation, ptosis, Horner's syndrome, muscle atrophy, neurologically impaired muscles, amyotrophic lateral sclerosis (ALS), motor neuron disease, any myopathy, multiple sclerosis, Parkinson's disease, myasthenia gravis, decrease in facial muscle tone, optionally ectropion, flaccid paralysis or weakness of any cause of any skeletal or smooth muscle, respiratory muscle weakness of any cause, including post-ventilator weakness, trauma-induced muscle weakness or poor posture caused by muscular flaccidity, pelvic floor muscle flaccidity or weakness, or nasal or upper respiratory flaccidity.

Other disorders that may be treated with a PEG-TeNT, a composition or method according to the disclosure include muscular atrophy, muscular dystrophy, decrease in muscle mass, nasal congestion, impotence, hair loss, hypotension, temporal mandibular joint syndrome, torticollis, neck pain, nerve regeneration within a muscle, migraine, headache, achalasia, obesity, spastic colon, anal fissures, tissues or organs affected by gastric acid, prostate hypertrophy, rhinorrhea, salivation, irritation of pulmonary mucosa, psoriasis, immune tolerance, immune reaction.

In the event that a disorder to be treated according to the disclosure is not a hypotonia disorder *per* se, increasing muscle tone by treating with a TeNT of the disclosure may alleviate a symptom of the disorder.

Cosmetic applications of PEG-TeNT may include tightening of the abdominal muscles, tightening of the pectoral muscles, tightening of the gluteus maximus, tightening of skeletal muscles, or treatment of facial droop caused by muscle flaccidity.

Smooth muscles, skeletal muscles, tissues or organs that may be treated with a PEG-TeNT, a composition or method according to the disclosure include upper oesophagus, oesophageal wall, oesophageal sphincter, lower oesophageal sphincter, anal sphincter, bladder, bladder sphincter, vaginal sphincter, pyloric sphincter, sphincter of Oddi, ileocaecal sphincter, pelvic floor muscles, vaginal wall muscles, prostate gland, submandibular gland, parotid gland, sublingual gland, minor salivary glands of the oral mucosa, vocal folds, facial muscles, mastication muscles, scalp muscles, chest muscles, back muscles, upper limb muscles, forearm muscles, lower limb muscles, hand muscles, foot muscles, stomach wall muscles, colon wall muscles, neck muscles, throat dilator muscles, masseter muscle, medial ptygeroid, lateral ptygeroid, geniohyoid, genioglossus, tensor veli palatine, levator veli palatini, stylopharyngeus, styloglossus, mylohyoid, stylohyoid, hyoglossus, diagastricus, sternocleidomastoid muscle, trapezius muscle, temporalis muscles, cricopharyngeus muscle, uterine muscle and cervix, gastric nerve supply, intranasal mucosa, pulmonary mucosa, skin, thymus, bone, coronary artery, pulmonary smooth muscle, and cardiac muscle.

### Compositions and administration

The composition of the disclosure may be a therapeutic composition or a cosmetic composition. That is, the composition may be used for therapy or cosmetic purposes.

As used herein, the term "therapeutic composition" or "cosmetic composition" refers to a composition comprising a TeNT that inhibits or treats hypotonia in the subject as described herein. The composition has been formulated for administration to a subject. In one embodiment, the composition is sterile. In one embodiment, the composition is pyrogen-free. The composition may comprise a pharmaceutically acceptable carrier. Preferably, the composition is manufactured according to Good Laboratory Practice (GLP) or Good Manufacturing Practice (GMP).

A TeNT of the disclosure may be administered at up to 10 mg/kg or more. A TeNT of the disclosure may be administered at about 1 fg/kg, about 5 fg/kg, about 10 fg/kg, about 50 fg/kg, about 100 fg/kg, about 500 fg/kg, about 1 pg/kg, about 5 pg/kg, about 10 pg/kg, about 50 pg/kg, about 100 pg/kg, about 500 pg/kg, about 1 ng/kg, about 2 ng/kg, about 3 ng/kg, about 4 ng/kg, about 5 ng/kg, about 6 ng/kg, about 7 ng/kg, about 8 ng/kg, about 9 ng/kg, about 10 ng/kg, about 11 ng/kg, about 12 ng/kg, about 13 ng/kg, about 14 ng/kg, about 15 ng/kg, about 16 ng/kg, about 17 ng/kg, about 18 ng/kg, about 19 ng/kg, about 20 ng/kg, about 30 ng/kg, about 40 ng/kg, about 50 ng/kg, about 60 ng/kg, about 70 ng/kg, about 80 ng/kg, about 90 ng/kg, about 100 ng/kg, about 200 ng/kg, about 300 ng/kg, about 400 ng/kg, about 500 ng/kg, about 600 ng/kg, about 700 ng/kg, about 800 ng/kg, about 900 ng/kg, about 1 pg/kg, about 5 pg/kg, about 10 pg/kg, about 50 pg/kg, about 100 pg/kg, about 500 pg/kg, about 1 mg/kg, or about 10 mg/kg. A TeNT of the disclosure may be administered within any range of any of the doses listed above.

A TeNT of the disclosure may be administered at up to 1000 IU/kg or more. A TeNT of the disclosure may be administered at about 0.1 IU/kg, about 0.2 IU/kg, about 0.3 IU/kg, about 0.4 IU/kg, about 0.5 IU/kg, about 0.6 IU/kg, about 0.7 IU/kg, about 0.8 IU/kg, about 0.9 IU/kg, about 1 IU/kg, about 2 IU/kg, about 3 IU/kg, about 4 IU/kg, about 5 IU/kg, about 6 IU/kg, about 7 IU/kg, about 8 IU/kg, about 9 IU/kg, about 10 IU/kg, about 11 IU/kg, about 12 IU/kg, about 13 IU/kg, about 14 IU/kg, about 15 IU/kg, about 16 IU/kg, about 17 IU/kg, about 18 IU/kg, about 19 IU/kg, about 20 IU/kg, about 30 IU/kg, about 40 IU/kg, about 50 IU/kg, about 60 IU/kg, about 70 IU/kg, about 80 IU/kg, about 90 IU/kg, about 100 IU/kg, about 200 IU/kg, about 300 IU/kg, about 400 IU/kg, about 500 IU/kg, about 600 IU/kg, about 700 IU/kg, about 800 IU/kg, about 900 IU/kg, about 1 000 IU/kg. A TeNT of the disclosure may be administered within any range of any of the doses listed above.

In a composition comprising two PEG-TeNTs, for example a composition comprising a first PEG-TeNT, wherein TeNT-HC is PEGylated (PEG-TeNT-HC), and a second PEG-TeNT wherein TeNT-LC is PEGylated and TeNT-c is PEGylated (PEG-TeNT-LC-c), the ratio of the first PEG-TeNT to the second PEG-TeNT may be varied. For example, the ratio of first PEG-TeNT to second PEG-TeNT may be about 1000:1, about 500:1, about 100:1, about 50:1, about 10:1, about 5:1, about 4:1, about 3:1, about 2:1, about 1:1; about 1:2, about 1:3, about 1:4, about 1:5, about 1:10, about 1:50, about 1:100, about 1:500, or about 1:1000.

A composition may comprise any combination of TeNTs and is not to be limited to a combination of PEG-TeNT-HC and PEG-TeNT-LC-c. A composition may comprise: PEG-TeNT-c and PEG-TeNT-HC; PEG-TeNT-c and PEG-TeNT-LC-c; PEG-TeNT-c and PEG-TeNT-LC-HC; PEG-TeNT-HC and PEG-TeNT-LC-HC; and PEG-TeNT-LC-c and PEG-TeNT-LC-HC. Also disclosed is a composition comprising: PEG-TeNT-c, PEG-TeNT-HC and PEG-TeNT-LC-c; PEG-TeNT-c, PEG-TeNT-HC and PEG-TeNT-LC-HC; PEG-TeNT-c, PEG-TeNT-LC-c and PEG-TeNT-LC-HC; PEG-TeNT-HC, PEG-TeNT-LC-c and PEG-TeNT-LC-HC; and PEG-TeNT-c, PEG-TeNT-HC, PEG-TeNT-LC-c and PEG-TeNT-LC-HC. In a composition, any TeNT may be substituted for, and any composition may further comprise, PEG-TeNT-LC, PEG-TeNT-LC-HN, and/or PEG-TeNT-HN.

In one embodiment, the composition further comprises an inactivated TeNT that acts as a decoy for anti-TeNT antibodies produced by prior exposure to TeNT, for example by vaccination. The inactivated TeNT may, relative to SEQ ID NO: 1, comprise: R1225K; R1225E; W1228A; W1288Y; W1288F; W1288L; R1225K and W1288A; R1225K and W1288Y; R1225E and W1288Y; R1225K and W1288F; R1225E and W1288F; R1225K and W1288L; R1225E and W1288L; R1225del; W1288del; or R1225del and W1288del; E270A; Y374A; E270A and Y374A; E270del; Y374del, or a combination thereof. In one embodiment, the second TeNT comprises an inactivated TeNT comprising R1225E, W1288A, E270A and Y374A.

In a composition comprising at least one PEG-TeNT and a decoy TeNT, the decoy TeNT will be in molar excess relative to the PEG-TeNT. For example, the ratio of decoy TeNT to the PEG-TeNT may be about 10⁶:1; 10⁵:1, 10⁴:1, 1000:1, about 500:1, about 400:1, about 300:1, about 200:1, about 100:1, about 90:1, about 80:1, about 70:1, about 60:1, about 50:1, about 40:1, about 30:1, about 20:1, about 10:1, about 9:1, about 8:1, about 7:1, about 6:1, about 5:1, about 4:1, about 3:1, about 2:1, or about 1:1.

A PEG-TeNT of the disclosure may be administered once, twice or three times per week, once, twice or three times per month, once, twice or three times per quarter, once, twice or three times per 6 months, or once, twice or three times per year.

The PEG-TeNT may be administered in a single dose, a split dose, or in multiple doses. Where a muscle exists as a pair, the PEG-TeNT may be administered unilaterally to one muscle of the pair or bilaterally to both muscles of the pair.

As an alternative to a combination comprising two or more PEG-TeNTs of the disclosure, such two or more PEG-TeNTs may be administered in combination sequentially or simultaneously.

The PEG-TeNT may be administered to a subject locally by any suitable method, for example by injection, surgical implantation, topical application, or intranasal administration. In one embodiment, the TeNT is administered intramuscularly by injection to the affected muscle.

The PEG-TeNT will be formulated, dosed, and administered in a fashion consistent with good medical practice. Factors for consideration in this context include the particular type of hypotonia being treated, the particular subject being treated, the clinical condition of the subject, the site of administration, the method of administration, the scheduling of administration, and other factors known to medical, including dental, practitioners. The therapeutically effective amount of the PEG-TeNT to be administered will be governed by such considerations.

Pharmaceutically acceptable carriers include water, buffered water, saline solutions such as, for example, normal saline or balanced saline solutions such as Hank's or Earle's balanced solutions, glycine, and hyaluronic acid.

The composition may be formulated for intramuscular administration. Compositions for intramuscular administration may comprise pharmaceutically acceptable sterile aqueous or non-aqueous solutions, dispersions, suspensions or emulsions as well as sterile powders for reconstitution into sterile injectable solutions or dispersions. Examples of suitable aqueous and non-aqueous carriers, solvents, diluents or vehicles include water, ethanol, polyols (such as glycerol, propylene glycol, polyethylene glycol), carboxymethylcellulose and mixtures thereof, vegetable oils (such as olive oil), injectable organic esters (e.g. ethyl oleate).

The composition may comprise penetration enhancers to enhance their delivery of TeNT. Penetration enhancers may include fatty acids such as oleic acid, lauric acid, capric acid, myristic acid, palmitic acid, stearic acid, linoleic acid, linolenic acid, dicaprate, reclineate, monoolein, dilaurin, caprylic acid, arachidonic acid, glyceryl 1-monocaprate, mono and di-glycerides and physiologically acceptable salts thereof.

The composition may further include chelating agents such as, for example, ethylenediaminetetraacetic acid (EDTA), citric acid, salicylates (e.g. sodium salycilate, 5-methoxysalicylate, homovanilate).

Also provided is an article of manufacture and/or a kit, comprising a container comprising the PEG-TeNT or composition comprising the PEG-TeNT. The container may be a bottle, vial or syringe comprising the PEG-TeNT or composition, optionally in unit dosage form. For example, the PEG-TeNT or composition may be in the form of an injectable solution in a disposable container, optionally a syringe. The article of manufacture and/or kit may further comprise printed instructions and/or a label or the like, indicating treatment of a subject according to the method disclosed herein.

The term "therapeutically effective amount" refers to an amount of PEG-TeNT effective to treat hypotonia in a subject.

The terms "treat", "treating" or "treatment" refer to both therapeutic treatment and prophylactic or preventative measures, wherein the aim is to prevent, reduce, or ameliorate hypotonia in a subject or slow down (lessen) progression of hypotonia in a subject. Subjects in need of treatment include those already with hypotonia as well as those in which hypotonia is to be prevented or ameliorated.

The terms "preventing", "prevention", "preventative" or "prophylactic" refers to keeping from occurring, or to hinder, defend from, or protect from the occurrence of hypotonia. A subject in need of prevention may be prone to develop hypotonia.

The term "ameliorate" or "amelioration" refers to a decrease, reduction or elimination of hypotonia.

Hypotonia may be quantified. Hypotonia may be quantified on a semi-quantitative scale, for example 0 to 5, where 0 represents absence, 1 to 4 represent identifiable increases in severity, and 5 represents maximum severity. Alternatively, hypotonia may be quantified as a binary event, i.e. presence or absence, 0 or 1. Other semi-quantitative scales will be readily apparent to the person skilled in the art. In another embodiment, hypotonia may be quantified on a quantitative scale, for instance using a force gauge.

Any quantification of hypotonia may be compared to a control, for example a healthy control subject not receiving a PEG-TeNT, an affected control subject receiving treatment for hypotonia, but not treated with a PEG-TeNT, or a population.

Treating hypotonia by administering a PEG-TeNT may be about a 1% decrease, about a 2% decrease, about a 3% decrease, about a 4% decrease, about a 5% decrease, about a 6% decrease, about a 7% decrease, about an 8% decrease, about a 9% decrease, about a 10% decrease, about a 20% decrease, about a 30% decrease, about a 40% decrease, about a 50% decrease, about a 60% decrease, about a 70% decrease, about an 80% decrease, about a 90% decrease, or about a 100% decrease in the hypotonia.

As used herein, the term "subject" may refer to a mammal. The mammal may be a primate, particularly a human, or may be a domestic, zoo, or companion animal. Although it is particularly contemplated that the PEG-TeNTs, compositions and method disclosed herein are suitable for medical treatment of humans, it is also applicable to veterinary treatment, including treatment of domestic animals such as horses, cattle and sheep, companion animals such as dogs and cats, or zoo animals such as felids, canids, bovids and ungulates.

Unless defined otherwise in this specification, technical and scientific terms used herein have the same meaning as commonly understood by the person skilled in the art to which this invention belongs and by reference to published texts.

It is to be noted that the term "a" or "an" refers to one or more, for example, "a TeNT" is understood to represent one or more TeNTs. As such, the terms "a" or "an", "one or more," and "at least one" may be used interchangeably herein.

In the claims which follow and in the description of the invention, except where the context requires otherwise due to express language or necessary implication, the word "comprise" or variations such as "comprises" or "comprising" is used in an inclusive sense, i.e. to specify the presence of the stated features, but not to preclude the presence or addition of further features in various embodiments of the invention.

The term "about" as used herein contemplates a range of values for a given number of +25% the magnitude of that number. In other embodiments, the term "about" contemplates a range of values for a given number of ±20%, ±15%, ±10%, ±5%, ±4%, ±3%, ±2%, or +1% the magnitude of that number. For example, in one embodiment, "about 3 grams" indicates a value of 2.7 grams to 3.3 grams (i.e. 3 grams ±10%), and the like.

Similarly, the timing or duration of events may be varied by at least 25%. For example, while a particular event may be disclosed in one embodiment as lasting one day, the event may last for more or less than one day. For example, "one day" may include a period of about 18 hours to about 30 hours. In other embodiments, periods of time may vary by ±20%, ±15%, ±10%, ±5%, ±4%, ±3%, ±2%, or ±1% of that period of time.

### EXAMPLES

### Example 1 - Preparation of PEG-TeNT-c (Figure 1A)

In this example, surface serine residues of TeNT-c will be mutated to surface cysteine residues to facilitate directed PEG conjugation at immunogenic epitopes to produce the molecule of Figure 1A. The mutations will be: S963C, S1041C, S1155C, and S1187C.

The gene for TeNT with surface serine to cysteine substitutions S963C, S1041C, S1155C, and S1187C will be synthesised by a commercial provider, for example Integrated DNA Technologies. The gene will be sub-cloned into the pRSET-A expression vector by restriction digestion so that 6x Histidine tag from the vector is added to the N-terminus of the mutant protein.

TeNT comprising S963C, S1041C, S1155C, and S1187C will be expressed, PEGylated and purified according to Example 5 to produce PEG-TeNT-c.

### Example 2 - Preparation of PEG-TeNT-HC (Figure 1B)

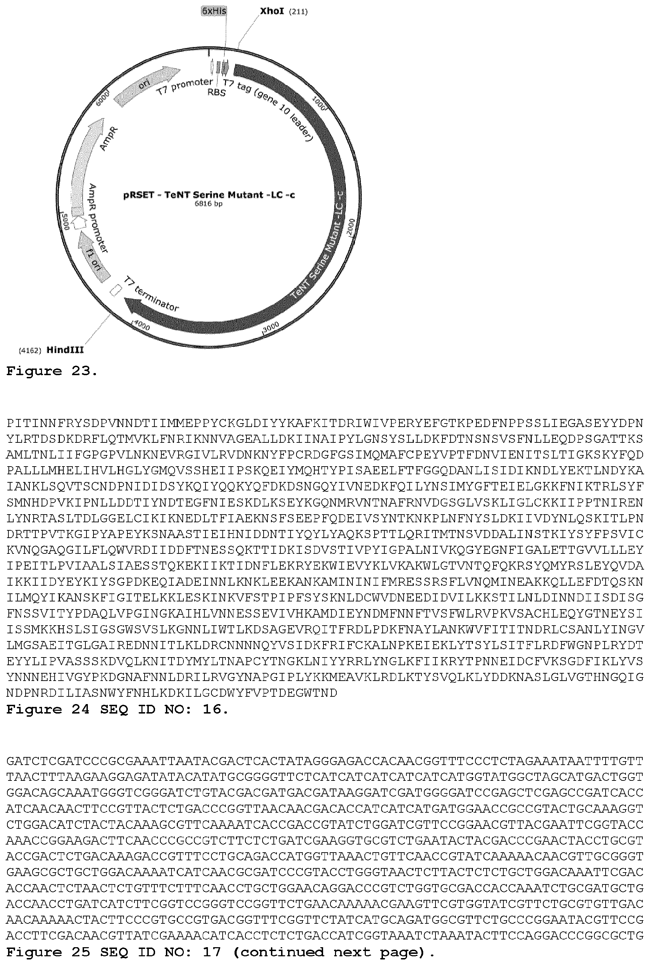

In this example, surface serine residues of TeNT-HC will be mutated to surface cysteine residues to facilitate directed PEG conjugation at immunogenic epitopes to produce the molecule of Figure 1B. The mutations will be: S600C, S963C, S1041C, S1155C, and S1187C (Figure 24 SEQ ID NO: 16).

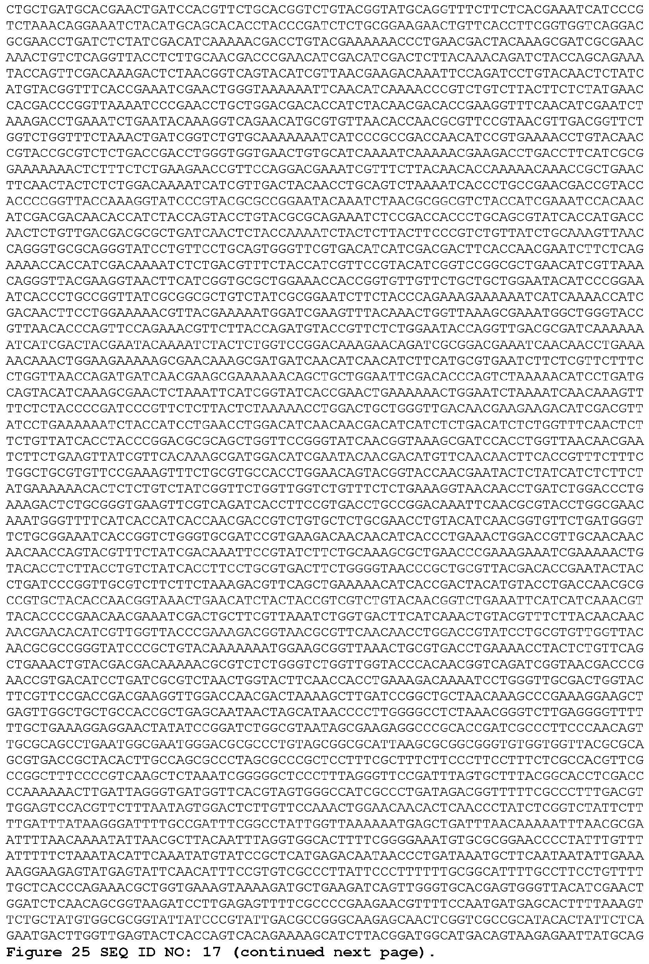

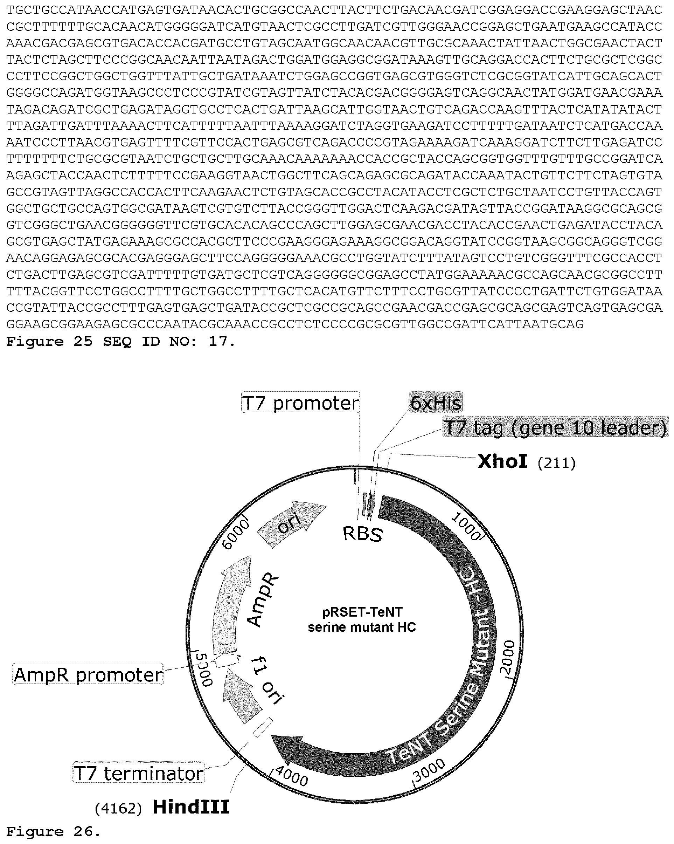

The gene for TeNT with surface serine to cysteine substitutions S600C, S963C, S1041C, S1155C, and S1187C will be synthesised by a commercial provider, for example Integrated DNA Technologies. The gene will be sub-cloned into the pRSET-A expression vector (Figure 25 SEQ ID NO: 17, Figure 26) by restriction digestion so that 6x Histidine tag from the vector is added to the N-terminus of the mutant protein.

TeNT comprising S600C, S963C, S1041C, S1155C, and S1187C will be expressed, PEGylated and purified according to Example 5 to produce PEG-TeNT-HC.

### Example 3 - Preparation of PEG-TeNT-LC-c (Figure 1C)

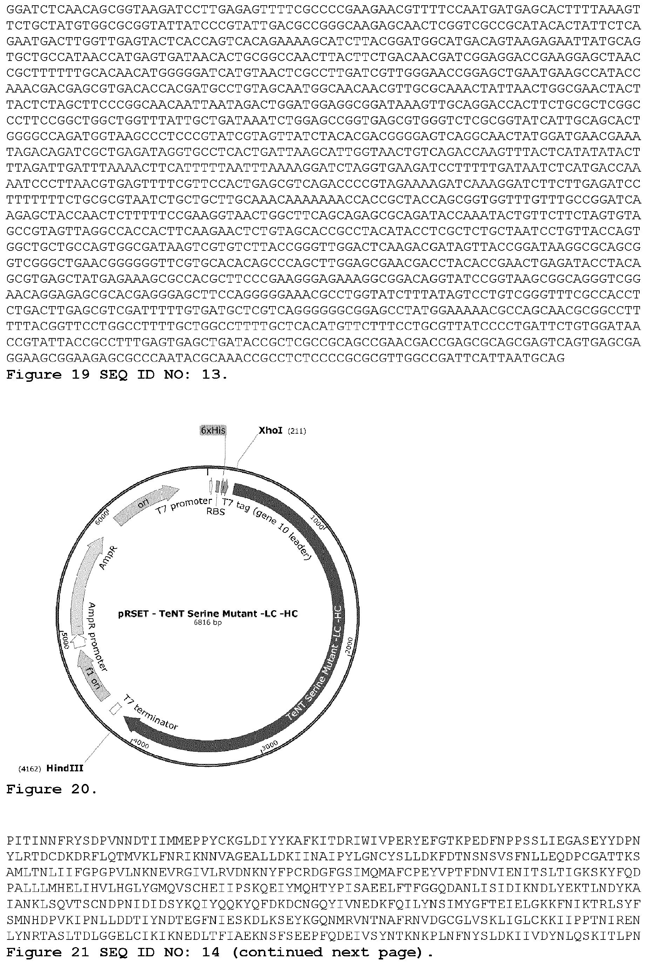

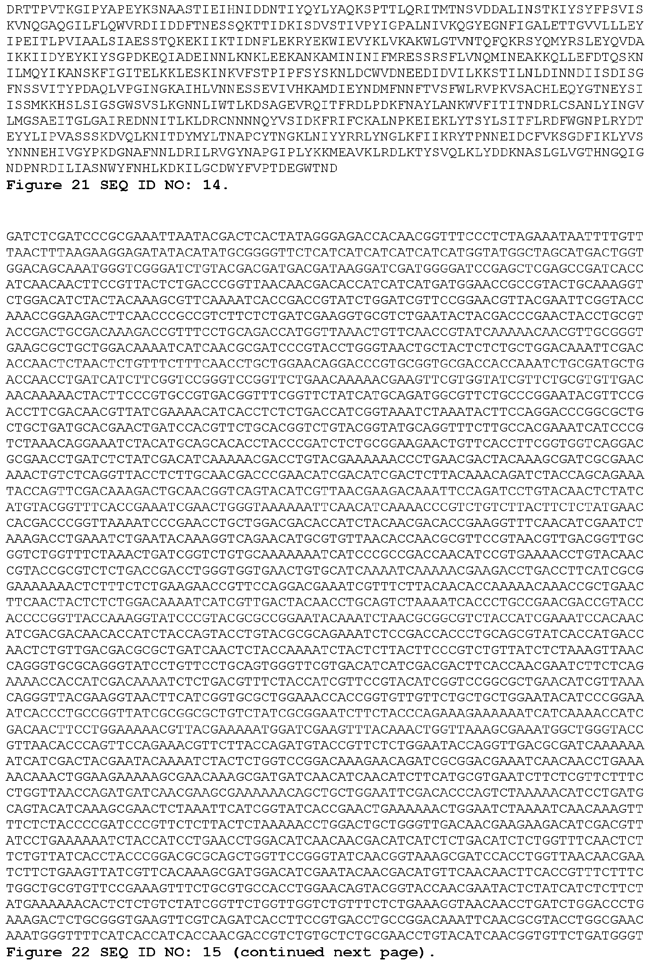

In this example, surface serine residues of LC and c were mutated to surface cysteine residues to facilitate directed PEG conjugation at immunogenic epitopes to produce the molecule of Figure 1C. The mutations were: S81C, S120C, S144C, S248C, S335C, S428C, S963C, S1041C, S1155C, and S1187C (Figure 21 SEQ ID NO: 14).

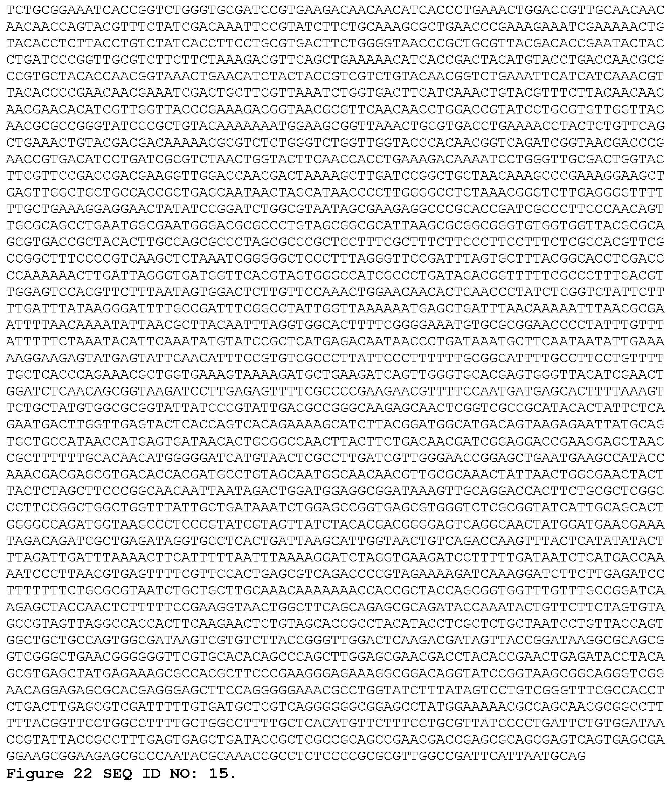

The gene for TeNT with surface serine to cysteine substitutions S81C, S120C, S144C, S248C, S335C, S428C, S963C, S1041C, S1155C, and S1187C in LC and c was synthesised by a commercial provider, Integrated DNA Technologies. The gene was sub-cloned into the pRSET-A expression vector (Figure 22 SEQ ID NO: 15, Figure 23) by restriction digestion so that 6x Histidine tag from the vector was added to the N-terminus of the mutant protein.

TeNT comprising S81C, S120C, S144C, S248C, S335C, S428C, S963C, S1041C, S1155C, and S1187C was expressed, PEGylated and purified according to Example 5 to produce PEG-TeNT-LC-c.

### Endotoxin removal

Endotoxin was removed from the TeNT-LC-c Serine mutant according to Example 4.

### PEG attachment to cysteine residues.

1. A molar excess of PEG - maleimide, with PEG of about 2 kDa, about 5 kDa, about 10 kDa or about 20 kDa, was combined with serine mutant TeNT-LC-c (0.5 - 2 mg/mL) in PBS at pH 6.5-7.5.
2. Attachment was performed at room temperature for 6 hours.
3. Excess PEG was removed by size exclusion chromatography.

### Trypsin digest activation of protein

Trypsin digestion for activation of the TeNT-LC-c Serine mutant was performed according to Example 4.

### Example 4 - Preparation of PEG-TeNT-LC-HC (Figure 1D)

### (method 1)

### Preparation of TeNT

1. *E. coli* BL21 DE3 pLysS strain was electrotransformed with pRSET-TeNT vector (Figures 3 and 4) and grown overnight on LB agar with selection antibiotic (ampicillin and chloramphenicol) at 37°C.
2. 200 mL of pre-induction broth was inoculated with 1 colony from the selection plate. The pre-induction broth pH 7.2-7.4 comprised: 1.2% Tryptone; 2.4% Yeast extract; 2% Glucose; 0.4% Glycerol; 17 mM KH₂PO₄; 72 mM K₂HPO₄; and selection antibiotics (ampicillin and chloramphenicol).
3. The culture was incubated overnight at 30°C with fast shaking.
4. The overnight culture was harvested by centrifugation at 4000 g for 10 minutes.
5. The pellet was resuspended in 200 mL expression broth pH 7.2-7.4, comprising: 1.2% Tryptone; 2.4% Yeast extract; 0.4% glycerol; 1 mM IPTG; 17 mM KH₂PO₄; 72 mM K₂HPO₄; 100 µg/mL ampicillin; and 10pM ZnCl₂.
6. The protein was expressed for 6 hours at 30°C with fast shaking.
7. The cells were harvested by centrifugation at 4500 g for 15 min and the pellet resuspended in 30 mL of TBS with 20 mM imidazole at pH 8.
8. The cells were lysed by sonication.
9. The cell lysate was cleared by centrifugation at 4500 g for 20 min and filtered through a 0.45 µm filter.
10. The protein was purified by His-tag affinity chromatography using the AKTA pure 25 FPLC system (GE).
11. The purified protein underwent buffer exchange to PBS using size exclusion chromatography followed by a second stage purification by gel filtration using AKTA pure 25 FPLC with Superdex 200 increase 10/300 GL column.

### Endotoxin removal

1. A 0.5 mL endotoxin removal spin column was equilibrated to room temperature.
2. The column bottom plug was removed, column cap loosened, column placed in a 15 mL tube then centrifuged at 500 g for 1 minute to remove solution from column. The solution was discarded.
3. The column bottom plug was replaced, column cap removed, 0.2N NaOH in 95% ethanol added to resin, column cap replaced, column inverted several times to resuspend resin, then incubated at room temperature for 1-2 h.
4. The column bottom plug was removed, column cap loosened, column place in a 15 mL tube then centrifuged at 500 g for 1 minute to remove solution from column. The solution was discarded.
5. The column bottom plug was replaced, column cap removed, endotoxin-free 2M NaCl added to resin, column cap replaced, and column inverted several times to resuspend resin.
6. The column bottom plug was removed, column cap loosened, column placed in a 15 mL tube then centrifuged at 500 g for 1 minute to remove solution from column. The solution was discarded.
7. The column bottom plug was replaced, column cap removed, endotoxin-free ultrapure water added to resin, column cap replaced, and column inverted several times to resuspend resin.
8. The column bottom plug was removed, column cap loosened, column placed in a 15 mL tube, and centrifuged at 500 g for 1 minute to remove solution from column. The solution was discarded.
9. The column bottom plug was replaced, column cap removed, endotoxin-free phosphate buffer added to resin, column cap replaced, and column inverted several times to resuspend resin.
10. The column bottom plug was removed, column cap loosened, column place in a 15 mL tube, and centrifuged at 500 g for 1 minute to remove solution from column. The solution was discarded.
11. The column was rinsed twice more with phosphate buffer and the eluate discarded.
12. The column bottom plug was replaced, column cap removed, sample applied to the resin, column cap replaced, and column inverted several times to resuspend resin.
13. The column was incubated with end-over-end mixing at 4°C for at least 1 h.
14. The column bottom plug was removed, column cap loosened, column placed in an endotoxin-free 15 mL tube, and centrifuged at 500 *g* for 1 minute to remove solution from column. The sample was retained.
15. The endotoxin removal procedure was repeated with a regenerated spin column until the endotoxin levels in the sample were at an equivalent or lower level so that all dosages would contain less than 5 EU units of endotoxin per kilogram of the subject.

### Preparation of PEG-TeNT-LC-HC.

1. In a total volume of 500 µL PBS pH 7.4, 3 pmol purified TeNT and 0.5 mmol mpeg-NHS(SC) (Nanocs) were combined with one of 2 kDa, 5 kDa, 10 kDa, 20 kDa, or 30 kDa PEG.
2. The sample was mixed at room temperature for 3 hours.
3. Excess PEG was removed by size exclusion chromatography.

### Trypsin digestion of protein into active form

1. 1 mg of the protein was dissolved in 0.5 mL digestion buffer, comprising 0.1 M NH₄HCO₃ buffer, pH 8.0 or 0.1 M Tris buffer pH 8.5.
2. 0.10 mL to 0.25 mL of Immobilized TPCK Trypsin was washed with 3 × 500 µL of digestion buffer. The gel was separated from the buffer after each wash by centrifugation.
3. The gel was resuspended in about 0.2 mL of the digestion buffer.
4. The Immobilized TPCK Trypsin was added to the protein sample.
5. The reaction mixture was incubated for 2 hours to 18 hours at 37°C in a rapidly shaking incubator.
6. The Immobilized TPCK Trypsin was separated by centrifugation.

### Example 5 - Preparation of PEG-TeNT-LC-HC (Figure 1D)

### (method 2)

In this example, surface serine residues of TeNT-LC-HC were mutated to surface cysteine residues (S to C mutant) to facilitate directed PEG conjugation at immunogenic epitopes. The TeNT mutations were: S81C; S120C; S144C; S248C; S335C; S428C; S600C; S963C; S1041C; S1155C; and S1187C, relative to SEQ ID NO: 1.

### Preparation of Serine mutant TeNT-LC-HC

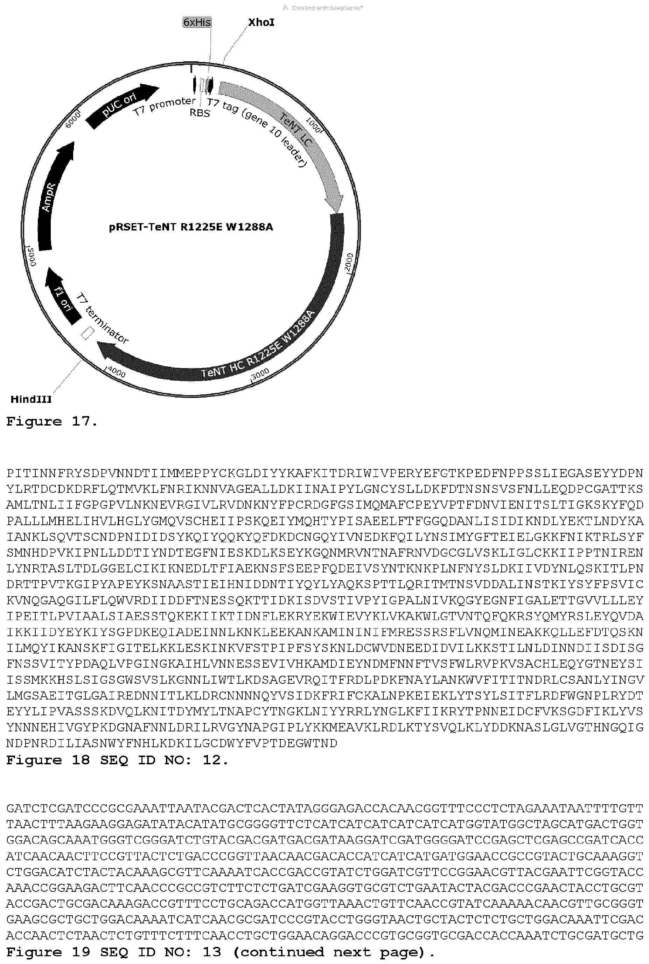

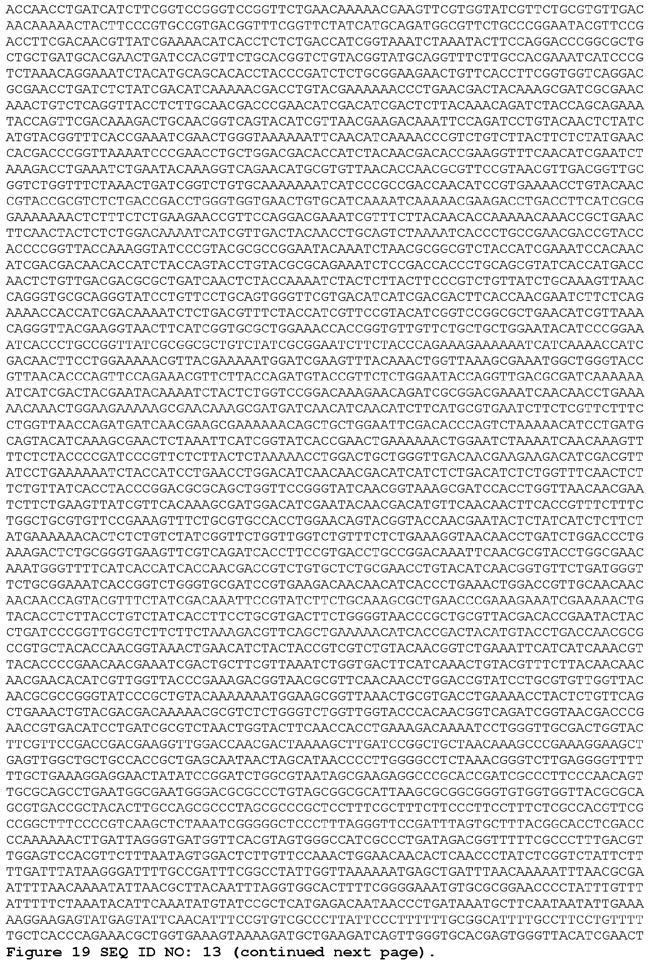

*E. coli* BL21 (DE3) *pLysS* strain was electrotransformed with vector pRSET-TeNT (Figures 19 and 20) encoding the amino acid sequence of Figure 18 (SEQ ID NO: 12) comprising the S to C mutations. TeNT-LC-HC comprising the S to C mutations was expressed and purified according to Example 4 with the addition of treatment by 0.5 mM DTT for 15 minutes between step 10 and 11.

### Endotoxin removal

Endotoxin was removed from the TeNT-LC-HC Serine mutant according to Example 4***.PEG* attachment to *cysteine residues.***
1. A molar excess of PEG - maleimide, with PEG of about 2 kDa, about 5 kDa, about 10 kDa or about 20 kDa, was combined with serine mutant TeNT-LC-HC (0.5 - 2 mg/mL) in PBS at pH 6.5-7.5.
2. Attachment was performed at room temperature for 6 hours.
3. Excess PEG was removed by size exclusion chromatography.

### Trypsin digest activation of protein

Trypsin digestion for activation of the TeNT-LC-HC Serine mutant was performed according to Example 4.

### Example 6 - Inactive Decoy TeNTs

In this example, a non-PEGylated, recombinant c with inactivating R1225E and W1288A amino acid substitutions, relative to SEQ ID NO: 1, in the ganglioside binding region was produced. The inactivated c was combined with an equimolar amount of non-PEGylated LC-HN to produce the inactive decoy TeNT. The resulting inactive TeNT will be used as a decoy for the antibody-based neutralising response in subjects vaccinated against tetanus toxoid.

### Preparation of c comprising R1225E W1288A

The gene for c with R1225E and W1288A was synthesised by a commercial provider, Integrated DNA Technologies. The gene was sub-cloned into the pRSET-A expression vector by restriction digestion so that 6x Histidine tag from the vector is added to the N-terminus of the mutant protein.

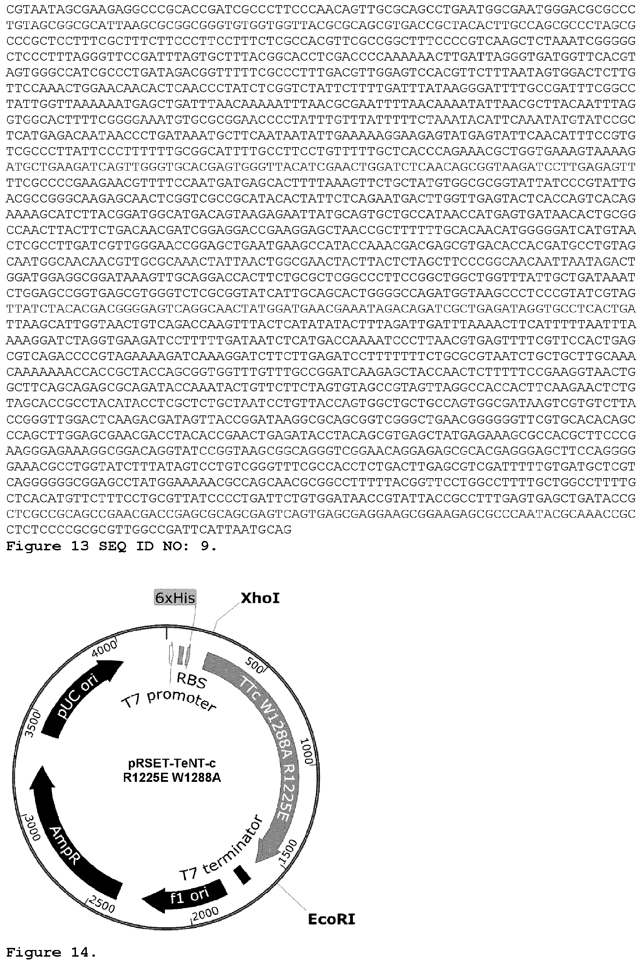

*E. coli* BL21 (DE3) *pLysS* strain was electrotransformed with pRSET-TeNT-c vector (Figure 13 (SEQ ID NO: 9) and Figure 14) encoding c comprising R1225E W1288A (Figure 12, SEQ ID NO: 8) and grown overnight on LB agar with selection antibiotics (ampicillin, chloramphenicol) at 37°C. c comprising R1225E W1288A was expressed and purified according to Example 4.

### Endotoxin removal

Endotoxin was removed from the purified protein according to Example 4.

### Preparation of LC-HN

*E. coli* BL21 (DE3) *pLysS* strain was electrotransformed with pRSET-TeNT-LC-HN vector (Figures 10 and 11) and grown overnight on LB agar with selection antibiotics (ampicillin, chloramphenicol) at 37°C. LC-HN was expressed and purified according to Example 4.

### Endotoxin removal

Endotoxin was removed from the purified protein according to Example 4.

### Preparation of LC-HN comprising E270A Y374A

*E. coli* BL21 (DE3) *pLysS* strain was electrotransformed with pRSET-TeNT-LC-HN vector encoding LC-HN comprising E270A Y374A and grown overnight on LB agar with selection antibiotics (ampicillin, chloramphenicol) at 37°C. LC-HN comprising E270A Y374A was expressed and purified according to Example 4.

### Endotoxin removal

Endotoxin was removed from the purified protein according to Example 4.

### Example 7 Inactive Decoy TeNTs

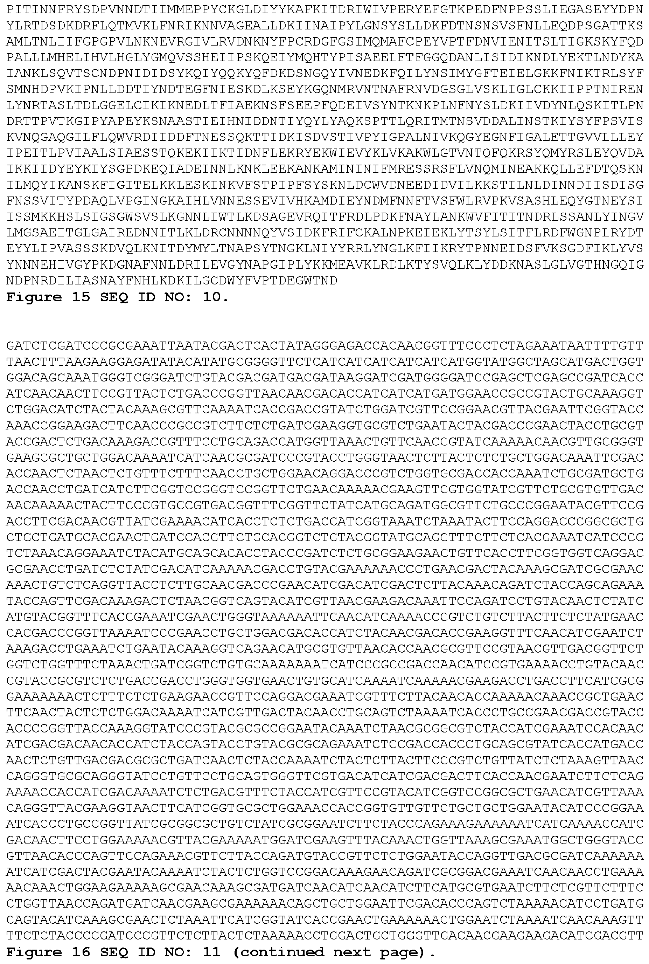

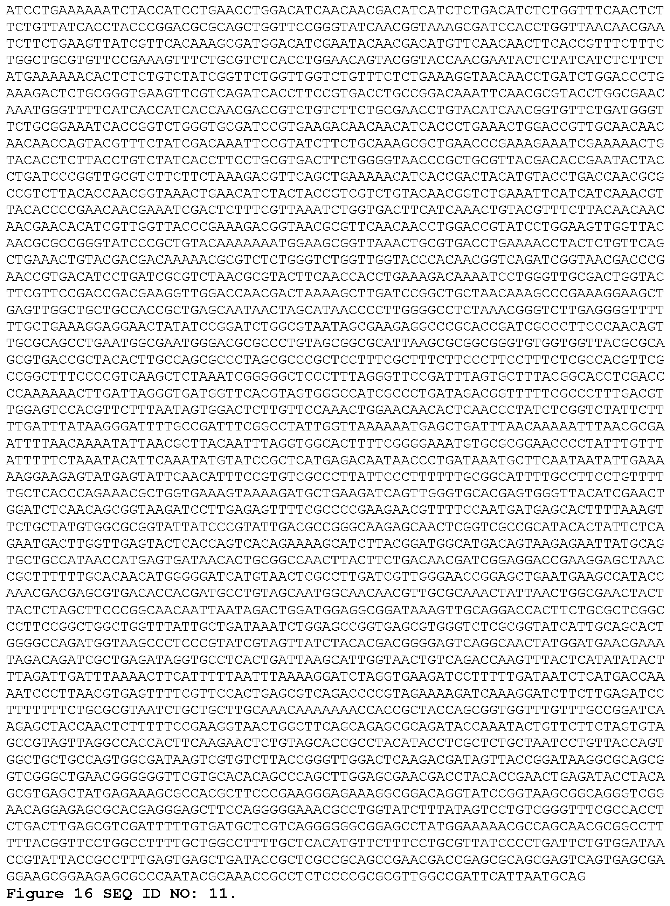

The gene for TeNT comprising R1225E and W1288A was synthesized by a commercial provider, Integrated DNA Technologies. The gene was be sub-cloned into the pRSET-A expression vector by restriction digestion so that 6x Histidine tag from the vector was added to the N-terminus of the mutant protein (Figure 15 SEQ ID NO: 10, Figure 16 SEQ ID NO: 11, and Figure 17).

The inactivated TeNT comprising R1225E and W1288A was expressed and purified according to Example 4.

### Endotoxin removal

Endotoxin was removed from the purified protein according to Example 4.

### Example 8 - PEG-TeNT analysis

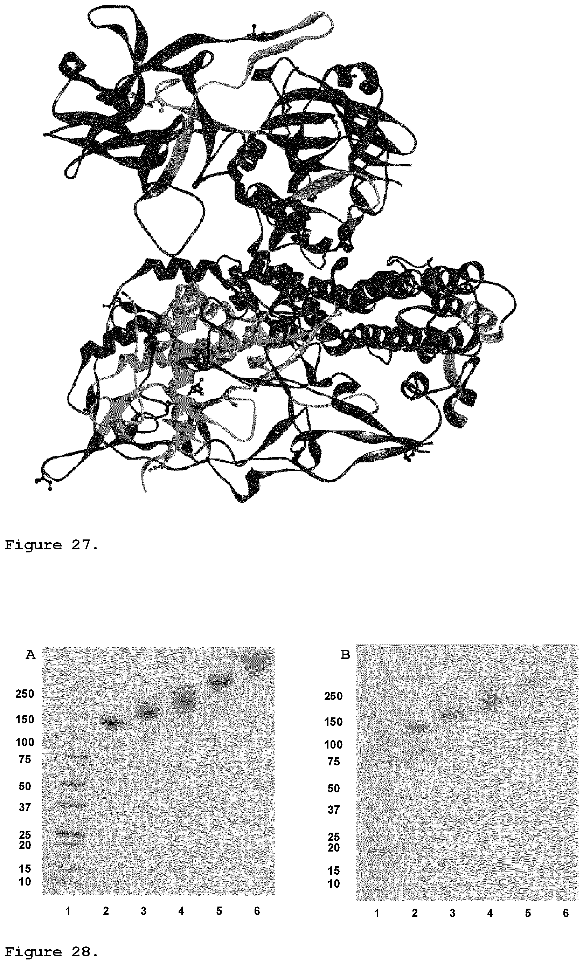

TeNT was prepared and PEGylated according to Example 4, then analysed by SDS-PAGE (Figure 28) and detected (A) using Coomassie blue and (B) by Western blot using polyclonal anti-TeNT antibodies. (B) shows that immunogenicity is proportional to PEG molecular weight.

### Example 9 - Reduced immunogenicity of PEG-TeNT versus TeNT

Four PEG-TeNTs each comprising a different molecular weight PEG (2 kDa, 5 kDa, 10 kDa and 20 kDa) were prepared and PEGylated according to Example 4. The PEG-TeNTs were then assayed by competitive ELISA against TeNT.

In the first assay (Figure 29A), TeNT was adsorbed to an ELISA plate. Adsorbed TeNT was then probed with a polyclonal anti-TeNT antibody pre-incubated with each of four concentrations (10 µg/mL, 1 µg/mL, 0.1 µg/mL and 0.01 µg/mL) of four PEG-TeNT antigens (2 kDa, 5 kDa, 10 kDa and 20 kDa). In this assay, higher responses (OD 450 nm) indicated greater affinity for TeNT and therefore reduced immunogenicity for the PEG-TeNT.

In the second assay (Figure 29B), each PEG-TeNT was adsorbed to a separate ELISA plate. Each adsorbed PEG-TeNT (2 kDa, 5 kDa, 10 kDa and 20 kDa) was then probed with a polyclonal anti-TeNT antibody pre-incubated with each of four concentrations (10 µg/mL, 1 µg/mL, 0.1 µg/mL and 0.01 µg/mL) of TeNT antigen. In this assay, lower responses (OD 450 nm) indicated greater affinity for TeNT and therefore reduced immunogenicity for the PEG-TeNT.

This example showed that anti-TeNT antibodies bound preferentially to TeNT and that PEGylated TeNTs have reduced immunogenicity relative to TeNT (i.e. non-PEGylated).

### Example 10 - Reduced immunogenicity of PEG-TeNT-LC-c Serine mutant versus TeNT-LC-c Serine mutant

TeNT-LC-c Serine mutant was prepared according to Example 5. Four samples of the TeNT-LC-c Serine mutant were PEGylated according to Example 5, each sample comprising a different molecular weight PEG (2 kDa, 5 kDa, 10 kDa and 20 kDa). The PEG-TeNT-LC-c Serine mutants were then assayed by competitive ELISA against TeNT-LC-c Serine mutant.

In the first assay (Figure 29C), TeNT-LC-c Serine mutant was adsorbed to an ELISA plate. Adsorbed TeNT Serine mutant was then probed with a polyclonal anti-TeNT antibody pre-incubated with each of four concentrations (10 µg/mL, 1 µg/mL, 0.1 µg/mL and 0.01 µg/mL) of four PEG-TeNT Serine mutant antigens (2 kDa, 5 kDa, 10 kDa and 20 kDa). In this assay, higher responses (OD 450 nm) indicated greater affinity for TeNT Serine mutant and therefore reduced immunogenicity for the PEG-TeNT Serine mutant.

In the second assay (Figure 29D), each PEG-TeNT-LC-c Serine mutant was adsorbed to a separate ELISA plate. Each adsorbed PEG-TeNT Serine mutant (2 kDa, 5 kDa, 10 kDa and 20 kDa) was then probed with a polyclonal anti-TeNT antibody pre-incubated with each of four concentrations (10 µg/mL, 1 µg/mL, 0.1 µg/mL and 0.01 µg/mL) of TeNT Serine mutant antigen. In this assay, lower responses (OD 450 nm) indicated greater affinity for TeNT Serine mutant and therefore reduced immunogenicity for the PEG-TeNT Serine mutant.

This example showed that anti-TeNT antibodies bound preferentially to TeNT-LC-c Serine mutant and that PEGylated TeNT-LC-c Serine mutants have reduced immunogenicity relative to TeNT-LC-c Serine mutant (i.e. non-PEGylated). s

### Example 11 - Reduced immunogenicity of PEG-TeNT versus TeNT

A competitive ELISA assay will be conducted in accordance with Example 9, except that the polyclonal antibody will be replaced by human serum collected from one or more subjects who have received a booster tetanus toxoid vaccination within the previous 12 months. Antibodies in the serum will show greater affinity for TeNT
(i.e. non-PEGylated TeNT) versus PEG-TeNTs.

### Example 12 - In vivo model

PEG-TeNT-LC-HC were prepared by attaching PEG (about 5 kDa, about 10 kDa or about 20 kDa) to the surface exposed lysine residues of recombinant TeNT accordng to Example 4.

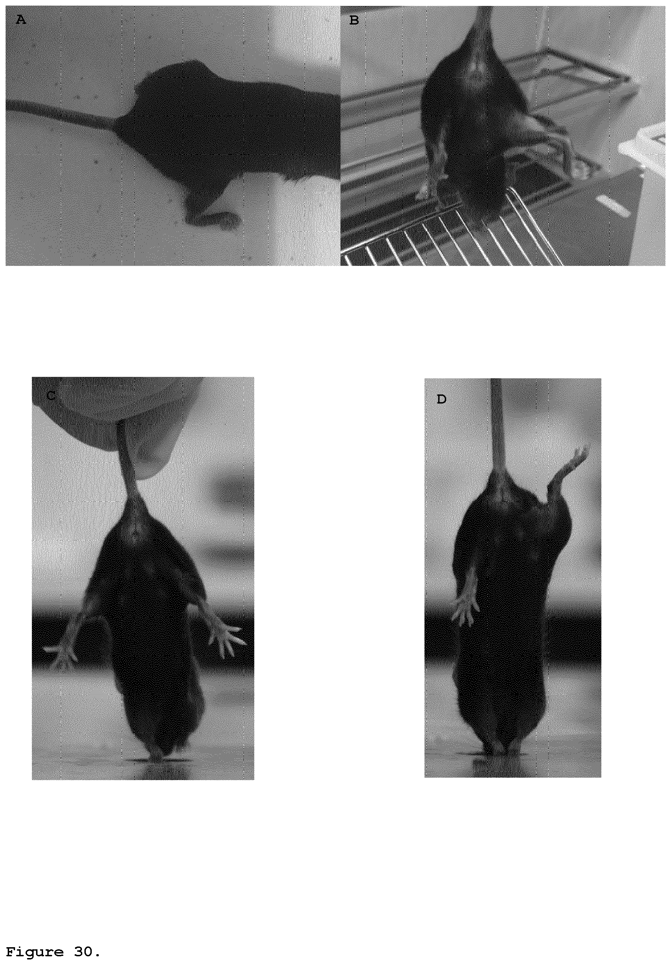

One or more units of PEG-TeNT-LC-HC in 15 µL of PBS was injected into the hind limb of female C57BL/6 mice. Each animal exhibited localised limb tetany within 48 hours of injection (Figure 30A and 30B).

### Example 13 - In vivo model

PEG-TeNT-LC-HC, with 5 kDa PEG attached to surface lysine residues of TeNT according to Example 4, was combined with c decoy, i.e. c inactivated by R1225E W1288A, at a molar ratio of 1:10. Fifteen microlitres of the composition in PBS, containing 5 ng of the PEG-TeNT, was injected into the muscle of the hind limb of female C57BL/6 mice. Each animal exhibited localised limb tetany within 48 hours, symptom development was indistinguishable from animals treated with the PEG-TeNT-LC-HC without the decoy present.

### Example 14.

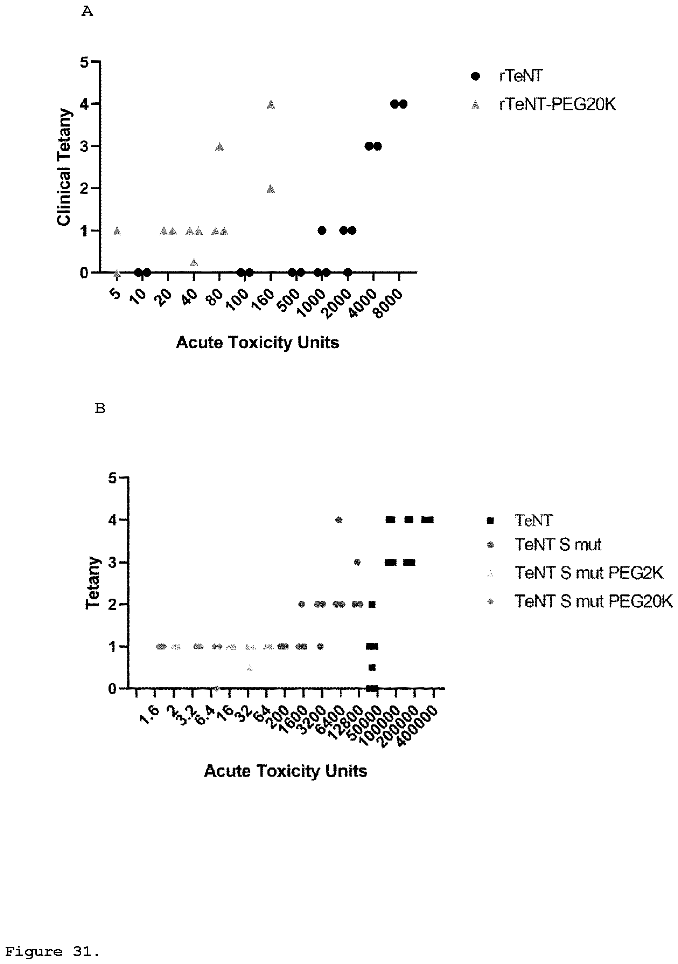

PEG-TeNT-LC-HC comprising 5 kDa, 10 kDa or 20 kDa PEG was administered at 50 - 500 000 ng/kg intramuscularly to the hind leg muscle of mice previously immunized with tetanus toxoid. Increased muscle contraction was observed in the injected muscle for up to 3 days, and was greater than the effect observed in mice administered the same units of TeNT. Figure 31 A reports results for PEG-TeNT-LC-HC 20kDa.

### Example 15.

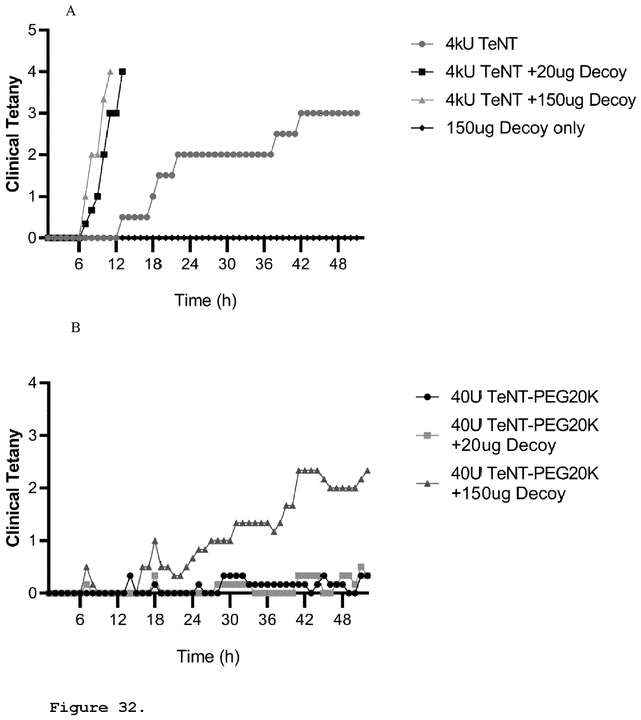

A composition comprising TeNT-LC-HC and a 10:1 or 100:1 molar excess of a decoy TeNT produced according to Example 6 was administered at 50 - 500 000 ng/kg intramuscularly to the hind leg muscle of mice previously immunized with tetanus toxoid. Increased muscle contraction was observed in the injected muscle for up to three days, and was greater than the effect observed in mice administered TeNT (Figure 32A).

### Example 16.

A composition comprising PEG-TeNT-LC-HC comprising 5 kDa, 10 kDa or 20 kDa PEG and a 10:1 or 100:1 molar excess of a decoy TeNT produced according to Example 6 was administered at 50 - 500 000 ng/kg intramuscularly to the hind leg muscle of mice previously immunized with tetanus toxoid. Increased muscle contraction was observed in the injected muscle for up to three days,and was greater than the effect observed in mice administered TeNT, and greater than the effect observed in mice administered PEG-TeNT only (Figure 32B).

### Example 17.

PEG-TeNT-LC-HC comprising 20 kDa PEG will be administered at 0.01 - 50 000 ng/kg intramuscularly to the left geniohyoid of a human subject previously vaccinated with tetanus toxoid. Increased muscle contraction will be observed in the injected muscle for up to 2 weeks, and will be greater than the effect observed in the right geniohyoid administered vehicle only of the same subject.

### Example 18.

A composition comprising PEG-TeNT-LC-HC comprising 20 kDa PEG and a 10:1 - 1000:1 molar excess of a decoy TeNT produced according to Example 6 will be administered at 50 ng/kg intramuscularly to the left geniohyoid of a human subject previously vaccinated with tetanus toxoid. PEG-TeNT-LC-HC comprising 20 kDa PEG will be administered at 50 ng/kg intramuscularly to the right geniohyoid of the same human subject.

Increased muscle contraction will be observed in both the left and right geniohyoids for up to 2 weeks, but will be greater in the left geniohyoid treated with the composition comprising PEG-TeNT-LC-HC and the decoy TeNT compared to the PEG-TeNT-LC-HC alone.

### Example 19.

Bulldogs of approximately 30 kg will be administered 25 - 50 000 ng/kg PEG-TeNT-c comprising 20 kDa PEG intramuscularly with the dose divided bilaterally to the left and right geniohyoid. Upon administration, obstructive sleep apnoea (OSA) will decrease in PEG-TeNT treated animals compared with animals treated with vehicle alone. The bulldogs will be observed weekly for OSA and the PEG-TeNT-c dose will be repeated as needed until efficacy decreases, as determined by a return of OSA comparable to animals treated with vehicle alone.

Thereafter, the bulldogs will be administered 25 - 50 000 ng/kg of PEG-TeNT-HC or 25 - 50 000 ng/kg PEG-TeNT-LC-c, each comprising 20 kDa PEG, divided bilaterally to the left and right geniohyoid. Upon administration, OSA will decrease in PEG-TeNT treated animals compared with animals treated with vehicle alone. The bulldogs will be observed weekly for OSA and the PEG-TeNT-HC and PEG-TeNT-LC-c dose will be repeated as needed until efficacy decreases, as determined by a return of OSA comparable to animals treated with vehicle alone.

Thereafter, the bulldogs will be administered 25 - 50 000 ng/kg PEG-TeNT-LC-HC, comprising 20 kDa PEG, divided bilaterally to the left and right geniohyoid. Upon administration, OSA will decrease in PEG-TeNT treated animals compared with animals treated with vehicle alone. The bulldogs will be observed weekly for OSA and the PEG-TeNT-LC-HC dose will be repeated as needed until efficacy decreases, as determined by a return of OSA comparable to animals treated with vehicle alone.

### Example 20

Discovery Studio was used to map TeNT epitopes recognised by major human antibody clonotypes, as identified by da Silva Antunes et al (2017) PloS One, 12(1), e0169086 and Palermo et al (2017) Biotechnology Journal, 12(10), 1700197, onto a 3-dimensional model of TeNT derived from crystallography data deposited in the protein data bank (accession ID PDB: 5N0B). Surface serine residues in or around the identified epitopes were identified for mutation to cysteine for subsequent PEGylation as set out in Examples 1 to 3 and 5.

### Example 21

One nanogram to 64 micrograms of a mixture of PEG-TeNT (5 kDa, 10 kDa, or 20 kDa branched or linear PEG attached to surface lysine or cysteine residues) and a 10-1000 molar excess of decoy TeNT will be injected intramuscularly into the hind leg muscle of mice vaccinated against tetanus toxoid. Localised tetany will be observed for a period of hours to months, greater than the effect observed in mice injected with native TeNT.

### Example 22

One nanogram to 64 micrograms of a mixture of PEG-TeNT (5 kDa, 10 kDa, or 20 kDa branched or linear PEG attached to surface lysine or cysteine residues) and a 10-1000 molar excess of decoy TeNT will be injected intramuscularly into the geniohyoid of a human vaccinated against tetanus toxoid. Localised tetani is observed for a period of weeks to months, greater than the effect observed in a human injected with native TeNT.

### Example 23

A British Bulldog with sleep disordered breathing was administered 0.001 - 10 IU/Kg of TeNT intramuscularly, with the dose divided bilaterally to the left and right geniohyoid. Upon administration, obstructive sleep apnoea (OSA) was observed to decrease significantly at the highest dose. The baseline respiratory disturbance index (RDI) score was 19.9 (interquartile range 5.45) decreasing to 13.2 (interquartile range 4.45) after treatment with 10 IU/Kg TeNT, determined to be significant compared to administration of a placebo by Wilcoxon signed rank test; by P=.043. The bulldog was observed four months post trials with the reduction in RDI maintained; median RDI=13.4, *P*=0.043; Wilcoxon Signed Ranks test.

### EMBODIMENTS

The following are further preferred embodiments of the present invention.
1. A tetanus neurotoxin (TeNT) or fragment thereof, comprising one or more surface serine to cysteine amino acid substitutions relative to SEQ ID NO: 1.
2. The TeNT of embodiment 1, wherein the substituted serine comprises one or more of S81C, S120C, S144C, S248C, S335C, S428C, S600C, S963C, S1041C, S1155C, and S1187C relative to SEQ ID NO: 1.
3. The TeNT of embodiment 1 or embodiment 2, wherein the substituted cysteine is conjugated to polyethylene glycol (PEG).
4. The TeNT of embodiment 3, wherein the PEG is about 2 kDa, 5 kDa, about 10 kDa, or about 20 kDa.
5. The TeNT of embodiment 3 or embodiment 4, wherein the PEG is linear or branched.
6. A TeNT or fragment thereof comprising, relative to SEQ ID NO: 1: R1225K; R1225E; W1288A; W1288Y; W1288F; W1288L; R1225K and W1288A; R1225E and W1288A; R1225K and W1288Y; R1225E and W1288Y; R1225K and W1288F; R1225E and W1288F; R1225K and W1288L; R1225E and W1288L; R1225del; W1288del; or R1225del and W1288del; E270A; Y374A; E270A and Y374; G270del; Y374del; or a combination thereof, wherein the TeNT or fragment is inactive.
7. A composition comprising: (i) a first PEGylated tetanus neurotoxin (PEG-TeNT) comprising tetanus neurotoxin (TeNT) conjugated to polyethylene glycol (PEG); and (ii) a second TeNT, wherein the first PEG-TeNT is not conjugated to the second TeNT.
8. The composition of embodiment 7, wherein the first PEG-TeNT comprises the TeNT of any one of embodiments 3 to 5.
9. The composition of embodiment 7 or embodiment 8, wherein the second TeNT is the TeNT of any one of embodiments 1 to 6.
10. A method for treating hypotonia, the method comprising administering to a subject: (i) a first PEGylated tetanus neurotoxin (PEG-TeNT) comprising tetanus neurotoxin (TeNT) conjugated to polyethylene glycol (PEG); and (ii) and a second TeNT.
11. Use of a first PEGylated tetanus neurotoxin (PEG-TeNT) comprising a tetanus neurotoxin (TeNT) conjugated to polyethylene glycol (PEG) in the manufacture of a medicament for treating hypotonia in a subject administered a second TeNT.
12. Use of a second TeNT in the manufacture of a medicament for treating hypotonia in a subject administered a first PEGylated tetanus neurotoxin (PEG-TeNT) comprising a tetanus neurotoxin (TeNT) conjugated to polyethylene glycol (PEG).
13. Use of: (i) a first PEGylated tetanus neurotoxin (PEG-TeNT) comprising a tetanus neurotoxin (TeNT) conjugated to polyethylene glycol (PEG); and (ii) a second TeNT in the manufacture of a medicament for treating hypotonia.
14. The composition of any one of embodiments 7 to 9, method of embodiment 10, or use of any one of embodiments 11 to 13, wherein the first PEG-TeNT or the second TeNT comprises a PEGylated TeNT light chain (LC), a PEGylated TeNT heavy chain (HC), or a PEGylated TeNT fragment c (c).
15. The composition, method or use of any one of embodiments 7 to 14, wherein the first PEG-TeNT or the second TeNT is PEG-TeNT-HC comprising a PEGylated HC, or is PEG-TeNT-LC-c comprising a PEGylated LC and a PEGylated c.
16. The composition, method or use of any one of embodiments 7 to 15, wherein the first PEG-TeNT is PEG-TeNT-HC, and the second TeNT is PEG-TeNT-LC-c.
17. The composition, method or use of any one of embodiments 7 to 16, wherein the second TeNT comprises an inactivated TeNT.
18. The composition, method or use of embodiment 17, wherein, relative to SEQ ID NO: 1, the inactivated TeNT comprises: R1225K; R1225E; W1228A; W1288Y; W1288F; W1288L; R1225K and W1288A; R1225E and W1288A; R1225K and W1288Y; R1225E and W1288Y; R1225K and W1288F; R1225E and W1288F; R1225K and W1288L; R1225E and W1288L; R1225del; W1288del; or R1225del and W1288del; E270A; Y374A; E270A and Y374; G270del; Y374del; or a combination thereof.
19. The method or use of any one of embodiments 9 to 18, wherein the subject is administered:
   a PEG-TeNT comprising PEGylated c (PEG-TeNT-c) until efficacy decreases; then
   a composition comprising PEG-TeNT-HC and PEG-TeNT-LC-c until efficacy decreases; then
   a PEG-TeNT comprising a PEGylated LC and a PEGylated HC (PEG-TeNT-LC-HC).
20. The method or use of any one of embodiments 9 to 19, wherein the hypotonia is obstructive sleep apnoea.
21. The composition of any one of embodiments 7 or 14 to 18, wherein the composition is a therapeutic composition.
22. A PEGylated tetanus neurotoxin (PEG-TeNT), comprising tetanus neurotoxin (TeNT) conjugated to polyethylene glycol (PEG).
23. The PEG-TeNT of embodiment 22, wherein the TeNT light chain (LC) is PEGylated, the TeNT heavy chain (HC) is PEGylated, or the TeNT fragment c (c) is PEGylated.
24. The PEG-TeNT of embodiment 22 or embodiment 23, wherein LC is PEGylated and HC is PEGylated (PEG-TeNT-LC-HC), or LC is PEGylated and c is PEGylated (PEG-TeNT-LC-c).
25. The PEG-TeNT of any one of embodiments 22 to 24, wherein PEG is conjugated to a lysine residue.
26. The PEG-TeNT of any one of embodiments 22 to 24, wherein PEG is conjugated to a cysteine residue, optionally wherein the cysteine residue is a surface serine to cysteine substitution relative to SEQ ID NO: 1.
27. The PEG-TeNT of any one of embodiments 22 to 26, wherein the PEG has a molecular weight of about 5 kDa, about 10 kDa or about 20 kDa.
28. A method for treating hypotonia, the method comprising administering to a subject the PEG-TeNT of any one of embodiments 22 to 27, wherein the PEG-TeNT is not conjugated to a second TeNT.
29. Use of the PEG-TeNT of any one of embodiments 22 to 27 in the manufacture of a medicament for treating hypotonia, wherein the PEG-TeNT is not conjugated to a second TeNT.
30. The method of embodiment 28 or use of embodiment 29, wherein the subject is administered a first PEG-TeNT comprising a PEGylated HC (PEG-TeNT-HC) and a second PEG-TeNT comprising a PEGylated LC and a PEGylated c (PEG-TeNT-LC-c).
31. The method or use of any one of embodiments 28 to 30, wherein the subject is administered:
   a PEG-TeNT comprising a PEGylated c (PEG-TeNT-c); and/or
   a first PEG-TeNT comprising a PEGylated HC (PEG-TeNT-HC) and a second PEG-TeNT comprising a PEGylated LC-c (PEG-TeNT-LC-c); and/or
   a PEG-TeNT comprising a PEGylated HC and a PEGylated LC (PEG-TeNT-LC-HC) .
32. The method or use of any one of embodiments 28 to 31, wherein treating comprises administering to the subject:
   a PEG-TeNT comprising PEGylated c (PEG-TeNT-c) until efficacy decreases; then
   a PEG-TeNT-HC and a PEG-TeNT-LC-c until efficacy decreases; then
   a PEG-TeNT comprising a PEGylated LC and a PEGylated HC (PEG-TeNT-LC-HC).
33. The method or use of any one of embodiments 28 to 32, wherein treating further comprises administering an inactivated TeNT.
34. The method or use of embodiment 33, wherein, relative to SEQ ID NO: 1, the inactivated TeNT comprises: R1225K; R1225E; W1288A; W1288Y; W1288F; W1288L; R1225K and W1288A; R1225E and W1288A; R1225K and W1288Y; R1225E and W1288Y; R1225K and W1288F; R1225E and W1288F; R1225K and W1288L; R1225E and W1288L; R1225del; W1288del; or R1225del and W1288del; E270A; Y374A; E270A and Y374; G270del; Y374del; or a combination thereof.
35. The method or use of any one of embodiments 28 to 34, wherein the hypotonia is obstructive sleep apnoea.
36. A kit comprising the TeNT of any one of embodiments 1 to 6, the composition of any one of embodiments 7, 14 to 18 or 21, or the PEG-TeNT of any one of embodiments 22 to 27.

## Claims

1. A tetanus neurotoxin (TeNT) or subdomain thereof for treating hypotonia in a subject, wherein the TeNT or subdomain thereof comprises:
a first PEGylated tetanus neurotoxin (PEG-TeNT) or PEGylated subdomain thereof comprising TeNT or a subdomain thereof conjugated to polyethylene glycol (PEG); or
a second TeNT or subdomain thereof comprising a second PEG-TeNT or PEGylated subdomain; or
a second TeNT comprising an inactivated TeNT.

2. The PEG-TeNT or subdomain thereof for treating hypotonia in a subject of claim 1, wherein the subject is to be administered:
the first PEG-TeNT or PEGylated subdomain thereof and the second PEG-TeNT or PEGylated subdomain thereof; or
the first PEG-TeNT or PEGylated subdomain thereof and the second TeNT comprising the inactivated TeNT.

3. The PEG-TeNT or subdomain thereof for treating hypotonia in a subject of claim 1 or claim 2, wherein the first PEG-TeNT or PEGylated subdomain thereof or the second TeNT or subdomain thereof comprises a PEGylated TeNT light chain (LC), a PEGylated TeNT heavy chain (HC), or a PEGylated TeNT fragment c (c).

4. The PEG-TeNT or subdomain thereof for treating hypotonia in a subject of any one of claims 1 to 3, wherein the first PEG-TeNT or PEGylated subdomain thereof or the second TeNT or subdomain thereof is PEG-TeNT-HC comprising a PEGylated HC, PEG-TeNT-LC-HC comprising PEGylated LC and a PEGylated HC, or is PEG-TeNT-LC-c comprising a PEGylated LC and a PEGylated c.

5. The PEG-TeNT or subdomain thereof for treating hypotonia in a subject of any one of claims 1 to 4, wherein, relative to SEQ ID NO: 1, the inactivated TeNT comprises: R1225K; R1225E; W1228A; W1288Y; W1288F; W1288L; R1225K and W1288A; R1225E and W1288A; R1225K and W1288Y; R1225E and W1288Y; R1225K and W1288F; R1225E and W1288F; R1225K and W1288L; R1225E and W1288L; R1225del; W1288del; R1225del and W1288del; E270A; Y374A; E270A and Y374A; E270del; Y374del; or a combination thereof.

6. The PEG-TeNT or subdomain thereof for treating hypotonia in a subject of any one of claims 1 to 5, wherein the first PEG-TeNT or PEGylated subdomain thereof or the second PEG-TeNT or PEGylated subdomain thereof comprises one or more surface serine to cysteine amino acid substitutions relative to SEQ ID NO: 1.

7. The PEG-TeNT or subdomain thereof for treating hypotonia in a subject of claim 6, wherein the serine to cysteine substitution comprises S81C, S120C, S144C, S248C, S335C, S428C, S600C, S963C, S1041C, S1155C, or S1187C, or any combination thereof, relative to SEQ ID NO: 1, optionally wherein the substituted cysteine is conjugated to PEG.

8. The PEG-TeNT or subdomain thereof for treating hypotonia in a subject of any one of claims 1 to 7, wherein the PEG is about 2 kDa, about 5 kDa, about 10 kDa, or about 20 kDa, optionally, wherein the PEG is linear or branched.

9. The PEG-TeNT or subdomain thereof for treating hypotonia in a subject of any one of claims 1 to 8, wherein the medicament is formulated to administer:
a PEG-TeNT comprising a PEGylated c (PEG-TeNT-c); and/or
a PEG-TeNT comprising a PEGylated HC (PEG-TeNT-HC) and a PEG-TeNT comprising a PEGylated LC-c (PEG-TeNT-LC-c); and/or
a PEG-TeNT comprising a PEGylated HC and a PEGylated LC (PEG-TeNT-LC-HC).

10. The PEG-TeNT or subdomain thereof for treating hypotonia in a subject of claim 9, wherein the medicament is formulated to administer:
a PEG-TeNT comprising PEGylated c (PEG-TeNT-c) until efficacy decreases; then
a PEG-TeNT-HC and a PEG-TeNT-LC-c until efficacy decreases; then
a PEG-TeNT comprising a PEGylated LC and a PEGylated HC (PEG-TeNT-LC-HC).

11. The PEG-TeNT or subdomain thereof for treating hypotonia in a subject of any one of claims 1 to 10, wherein the hypotonia is obstructive sleep apnoea.

12. A composition comprising: (i) a first PEGylated tetanus neurotoxin (PEG-TeNT) or PEGylated subdomain thereof comprising tetanus neurotoxin (TeNT) or a subdomain thereof conjugated to polyethylene glycol (PEG); and (ii) a second TeNT or subdomain thereof, wherein the second TeNT or subdomain thereof is a second PEG-TeNT or PEGylated subdomain or an inactivated TeNT.

13. The composition of claim 12, wherein the first PEG-TeNT or PEGylated subdomain thereof or the second PEG-TeNT or PEGylated subdomain thereof comprises one or more surface serine to cysteine amino acid substitutions relative to SEQ ID NO: 1, and wherein the substituted cysteine is conjugated to PEG, optionally wherein the PEG is about 2 kDa, about 5 kDa, about 10 kDa, or about 20 kDa, optionally wherein the PEG is linear or branched.
